# EUROPEAN PATENT SPECIFICATION

(11) **EP 0 967 923 B1**
(45) Date of publication and mention of the grant of the patent: **22.11.2006**
(21) Application number: 99904368.0
(22) Date of filing: 27.01.1999
(51) Int. Cl.: A61B 17/04

(54) **PROTECTIVE SHEATH FOR TRANSVAGINAL ANCHOR IMPLANTATION DEVICE**
SCHUTZHÜLLE FÜR EINE TRANSVAGINALE ANKEREINSETZVORRICHTUNG
GAINE DE PROTECTION POUR DISPOSITIF TRANSVAGINAL D'IMPLANTATION D'ANCRAGES

(30) Priority: 27.01.1998 US 72641 P
(43) Date of publication of application: 05.01.2000
(73) Proprietor: Boston Scientific Limited, St. Michael, West Indies (BB)
(72) Inventor: GELLMAN, Barry, N., North Easton, MA 02356 (US); SAUVAGEAU, David, J., Methuen, MA 01844 (US); MORIN, Armand, A., Berkeley, MA 02779 (US); BRENNEMAN, Rodney, Dana Point, CA 92629 (US)
(74) Representative: Wilson, Alan Stuart
(86) International application number: PCT/US1999/001805
(87) International publication number: WO 1999/037216

(56) References cited:
- WO-A-97/30638
- WO-A-98/19606
- US-A- 5 681 352

## Description

### Technical Field

This invention relates to a protective sheath for a bone anchor implantation device. The bone anchor implantation device is used in maintaining or improving urinary continence.

### Background Information

Urinary incontinence, the inability to control urination from the bladder, is a widespread problem in the United States and throughout the world. Urinary incontinence affects people of all ages and can severely impact a patient both physiologically and psychologically.

In approximately 30% of the women suffering from urinary incontinence, incontinence is caused by intrinsic sphincter deficiency (ISD), a condition in which the valves of the urethral sphincter do not properly coapt. In approximately another 30% of incontinent women, incontinence is caused by hypermobility, a condition in which the muscles around the bladder relax, causing the bladder neck and proximal urethra to rotate and descend in response to increases in intraabdominal pressure. Hypermobility may be the result of child delivery or other conditions which weaken, stretch or tear the muscles. In an additional group of women with urinary incontinence, the condition is caused by a combination of ISD and hypermobility.

In males, urinary incontinence may be the consequence of post radical prostatectomy, which can destroy the valves of the urethral sphincter.

In addition to the conditions described above, urinary incontinence has a number of other causes, including birth defects, disease, injury, aging, and urinary tract infection.

Numerous approaches for treating urinary incontinence are available. In one procedure, referred to as bladder neck stabilization (BNS), sutures are placed around the muscles on either side of the urethra and affixed to the rectus fascia or pubic bone and tensioned to treat hypermobility. Other procedures which treat both hypermobility and intrinsic sphincter deficiency (ISD) involve the placement of a sling under the urethra/bladder which compresses the sphincter while simultaneously acting as a stabilizer of the bladderneck (preventing excessive downward mobility). The bone anchors which support the sling sutures may be inserted into rectus fascia or various locations on the pubis bone to provide a non-moveable anchoring method. Examples of bone anchor implantation devices are disclosed in WO 97/30638 (Influence, Inc.). WO-A-9 819 606 discloses a bone anchor implantation device comprising a hook-shaped shaft having a first and a second end, a bone anchor releasable engaged to one end of the shaft and a protective cap.

### Summary of the Invention

The present invention is as defined in the appended set of claims and generally relates to devices for inserting anchors, such as bode anchors, into a bone or tissue and more particularly to a protective sheath or cap for isolating the bone anchor to prevent both accidents with the sharp bone anchor before it is inserted into a target site and contamination of the target site by insertion of the bone anchor therethrough.

Bone anchors are often attached into bones in order to provide support for a "sling" useful in improving or maintaining a patient's urinary incontinence. In one procedure, a suture-carrying anchor is driven through the vaginal wall and into the posterior portion of the pubic bone or symphysis pubic, and the suture(s) attached to the bone anchor(s) extend through the vaginal wall and may be attached to the endopelvic fascia, the vaginal wall, a sling, or other material to stabilize and/or slightly compress the urethra thereby improving the patient's urinary incontinence. The present invention effectively addresses concerns in affixing an anchor to bone or tissue.

The present invention is directed to a protective sheath for the bone anchor. The protective sheath prevents accidents with the sharp bone anchor tip before insertion into the target site, and it prevents infection of the pubic bone. The protective sheath prevents exposure and accidental puncture of the surgeon's gloves as well as premature insertion into unintended tissue in the patient. It also provides a sterile barrier around the bone anchor. The protective sheath shields the bone anchor from contacting microorganisms in the vagina and area surrounding the implantation site during insertion. The protective sheath of the present invention ensures that the bone anchor implants into the bone implantation site free from contamination and thus prevents the occurrence of biological complications.

One aspect of the present invention is a bone anchor implantation device comprising an elongated member having a first end and a second end. A bone anchor is releasably engaged to the elongated member in the vicinity of the first end. A protective sheath comprising a balloon is mounted over the bone anchor. The protective sheath can be axially movable relative to the bone anchor such that the bone anchor is exposed from the sheath as the bone anchor is pressed into a bone by the elongated member. The protective sheath may be a gelatin structure that encapsulates or covers the bone anchor prior to implantation. The balloon or thin film can be hermetically sealed around the bone anchor, but in any case the balloon isolates the bone anchor from contact with tissue and prevents contamination prior to implantation of the bone anchor. The balloon is perforated by the bone anchor as the bone anchor is pressed into the bone by the elongated member or shaft. The balloon may be made of a variety of materials such as plastic, thermoplastic, elastromers, PET, PETG, rubber, vinyl, latex, or silicone. In one preferred embodiment, the balloon is made of latex. The balloon can also be made of a biodegradable material In another preferred embodiment, the balloon comprises a polymer such as a synthetic polymer. Nonlimiting examples of useful polymers include the flowing: polyglycolic acid (PGA), polyactic acid(PLA), poly (dioxanone) (PDO), poly (1-lactide) (LPLA), poly (dl-lactide) (DLPLA), poly (glycolide-co-trimethylene carbonate) (PGA-TMC), poly (1-lactide-co-glycolide) (PGA-LPLA), poly (dl-laetide-co-glycolide) (PGA-DLPLA), poly (1-lactide-co-dl-lactide) (LPLA-DLPLA), poly(glycohdo-co-trimethylene carbonate-codioxanone) (PDO-PGA-TMC), poly(ε-caprolactone), Poly(dioxanono)(a palyether-ester), poly (lactide-co-glycotide), Pcly(SA-HDA anhydride), poly(orthoester), and polyglyconate. The protective sheath can also take the form of a gelatin structure (similar to a pill capsule).

In some embodiments, the protective sheath (e.g. balloon or gelatin structure) can contain an antibiotic which is released when the sheath is perforated by the bone anchor. The antibiotic prevents infection at the site where the bone anchor is pressed into the bone. Nonlimiting examples of antibiotics which can be used include the following: nafcillin, aminogylcoside, ciprofloxin, clindamcin, piperacillin/tazobactum, ampicillin/sulbactum, aminoglcoside, vancomycin, cephalosporin, TMP/SMX, ampicillin, gentaminicin, tobramycin, and ciprofloxacin. Those skilled in the art will appreciate that there are numerous ways to insert the antibiotic into the balloon or the gelatin structure. In one embodiment, the bone anchor implantation device has a port which extends from the first end to the second end of the shaft into the balloon or gelatin structure. Antibiotics can be inserted into the protective sheath through a port.

In general, in another aspect, the invention features a bone anchor implantation device which has a spring attached to the sheath within the balloon which retracts when the sheath is pressed against the bone by the shaft, thereby causing the bone anchor to perforate the balloon and implant into the bone. The spring element may be an open-coiled helical spring which surrounds the bone anchor. The spring element retracts when pressure is applied to the sheath causing the bone anchor to puncture the balloon.

In some embodiments, the shaft of the bone anchor implantation device can have a hollow section which accommodates one or more sutures coupled to the releasably engaged bone anchor. Also, the shaft preferably is hook shaped.

A method for inserting such a bone anchor that is releasably engaged to such a bone anchor implantation device can include the steps of locating a bone anchor implantation site on the bone and applying a retrograde force to the bone anchor to implant the bone anchor into the bone or to retract the spring to cause the bone anchor to perforate the sheath and implant into the bone.

The foregoing and other objects, aspects, features, and advantages of the invention will become more apparent from the following description and from the claims.

### Brief Description of the Drawings

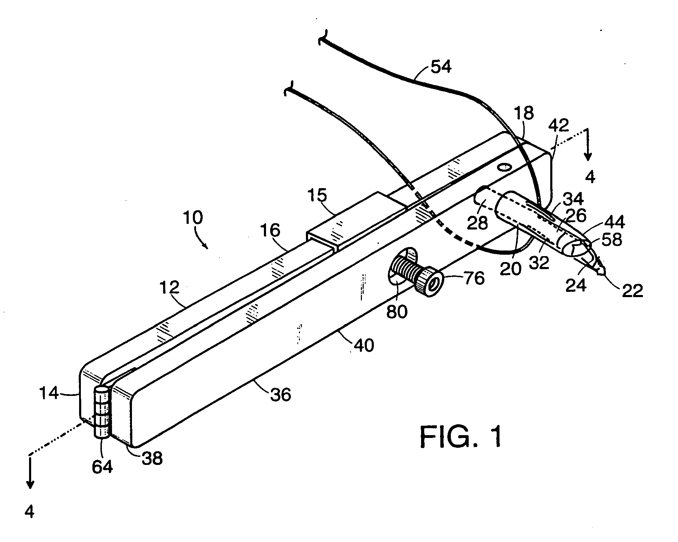

In the drawings, like reference characters generally refer to the same parts throughout the different views. Also, the drawings are not necessarily to scale, emphasis instead generally being placed upon illustrating the principles of the invention. Figures 1-12 relate to embodiments not forming part of the present invention.
Figure 1 is a plan view of one anchor implantation device.
Figure 2 is an exploded view of the anchor implantation device.
Figure 3 is a cross-sectional view of the distal end of the cannula showing the protective cap therein taken along line 3-3 of Figure 2.
Figure 4 is a cross-sectional view of the bone anchor implantation device of Figure 1 taken along line 4-4 of Figure 1.
Figure 5 is a cross-sectional view of the bone anchor implantation in its locked configuration.
Figure 6 is a plan view of an alternate embodiment of the bone anchor implantation device having a keyhole-shaped bore in the second handle.
Figure 7 is a cross-sectional view taken along line 7-7 of the alternate embodiment shown in Figure 6 locked in the position in which the inserter shaft is fully extended from the cannula.
Figure 8 is a cross-sectional view of the alternate embodiment of Figure 6 locked in the position in which the inserter shaft is fully retracted within the cannula.
Figure 9 is a cross-sectional view of the distal end of the inserter shaft in the cannula showing location of the bone anchor implantation site by sliding the cannula along the endopelvic fascia.
Figure 10 is a cross-sectional view of the distal end of the inserter shaft and the cannula showing the inserter shaft penetrating the protective cap near the distal end of the cannula.
Figure 11 is a cross-sectional view of the distal ends of the inserter shaft in the cannula showing the bone anchor being drive into the bone.
Figure 12 shows the bone anchor with sutures extending therefrom after implantation into the bone.
Figure 13 is a side view of a bone anchor implantation device having a hooked shaft.
Figure 14 is an enlarged side view of a distal portion of the bone anchor implantation device taken along line 14-14 of Figure 13 showing the internal structure of the bone anchor mount.
Figure 15 is a perspective view of the bone anchor mount.
Figure 16 is a cross sectional view of the bone anchor mount of Figure 15 taken along line 16-16.
Figure 17 is a schematic view showing the interior structure of the handle an alternate embodiment of the bone anchor implantation device inserted into the vagina with the proximal end of the second telescoping cylinder contacting the pubic bone.
Figure 18 is an enlarged view of the shaft of the alternate embodiment of the bone anchor implantation device illustrated in Figure 17.
Figure 19 is a cross sectional view of the shaft of the bone anchor implantation device shown in Figure 18 taken along line 19-19 of Figure 18.
Figure 20 is a schematic view showing the interior structure of the handle of an alternate embodiment of the bone anchor implantation device illustrated in Figure 17 inserted into the vagina showing the implantation of a bone anchor into the pubic bone and the compression of the spring.
Figure 21 is a side view of the bone anchor implantation device of Figure 13 showing a protective sheath contacting the pubic bone.
Figure 22 is a side view of the bone anchor implantation device of Figure 13 showing the bone anchor implanted into the pubic bone.
Figure 23 is a cross sectional view of the bone anchor mount and protective sheath when the protective sheath is containing the pubic bone.
Figure 24 is a cross sectional view of the bone anchor mount and the protective sheath when the bone anchor is being implanted into the pubic bone.
Figure 25 is a side view of a bone anchor implantation device having a hooked-shaped shaft and a balloon.
Figure 26a is a perspective view of the bone anchor and a balloon.
Figure 26b is a perspective view of a bone anchor and a gelatin structure.
Figure 27 is a schematic view showing the interior structure of the handle of an alternate embodiment of the bone anchor implantation device with balloon inserted into the vagina with the proximal end of the second telescoping cylinder contacting the pubic bone.
Figure 28 is a side view of the bone anchor implantation device of Figure 25 showing a balloon contacting the pubic bone.
Figure 29 is a side view of the bone anchor implantation device of Figure 25 showing the bone anchor implanted into the pubic bone.
Figure 30 is a side elevational view of a bone anchor.
Figure 31 is a side elevational view of the spear member of bone anchor shown in Figure 30.
Figure 32 is a rear elevational view of the spear member of the bone anchor shown in Figure 30.
Figure 33 is an enlarged sectional view of a shaft of the spear member of the bone anchor taken along the lines 4--4 of Figure 31.
Figure 34 is a side elevational view of a collar member of the bone anchor shown in Figure 30.
Figure 35 is a rear elevational view of the collar member of the bone anchor shown in Figure 30.
Figure 36 is a side elevational view of the collar member of the bone anchor taken along the direction of the lines 7--7 of Figure 35.
Figure 37 is a sectional view of the collar member of the bone anchor taken along the lines 8--8 of Figure 35.
Figure 38 is a perspective view of the bone anchor tip on a bone anchor implantation device having a balloon with a spring element.
Figure 39 is a side view of a bone anchor implantation device with a telescoping sheath.
Figure 40a is an enlarged view of the telescoping sheath of Figure 39 with the telescoping sheath in an expanded position.
Figure 40b is an enlarged view of the telescoping sheath of Figure 39 with the telescoping sheath in a retracted position.
Figure 41 a is a view of a bone anchor implantation device with a balloon sheath.
Figure 41b is an enlarged view of the balloon sheath on bone anchor implantation device of Figure 41a.
Figure 42a is a view of a bone anchor implantation device with a latex sheath and a spring element.
Figure 42b is another view of the bone anchor implantation with a latex sheath and a spring element.
Figure 43 is a view of a bone anchor implantation device with a caplet sheath.
Figure 44 is a view of a bone anchor implantation device that has a lumen that can hold one or more sutures.
Figure 45 is a view of a bone anchor implantation device of Figure 39 inserted into the vagina showing the implantation of a bone anchor into the pubic bone.

### Description

The present invention relates to a device for affixing a bone anchor to a bone. More particularly, the invention relates to a protective sheath for protecting the bone anchor from contacting tissue during implantation and thereby preventing contamination of the bone anchor. It can be used in methods for improving or maintaining a patient's urinary continence in which bone anchors are inserted transvaginally into the posterior portion of the pubic bone or symphysis pubis and devices for use in such methods. As used herein, the terms "transvaginally" or "transvaginal access" refer to access through the vaginal introitus or from within the vagina, as opposed to access from the patient's abdominal side.

As will be described in more detail below, the devices of the present invention drive a bone anchor through the vaginal wall and into the posterior portion of the pubic bone or symphysis pubis. The public bone may also be accessed through a suprapublic bone incision. Preferably, at least one bone anchor is driven into the pubic bone on either side of the urethra. However, one of skill in the art will appreciate that a single bone anchor may also be used. The sutures attached to the bone anchors extend through the vaginal wall and may then be attached to the endopelvic fascia, the vaginal wall, a sling, or other material to stabilize and/or slightly compress the urethra, thereby improving or maintaining the patient's urinary continence.

### Two Handle Bone Anchor Implantation Device

In one embodiment, the anchor implantation device has a first handle having an inserter shaft attached thereto. The inserter shaft is adapted to releasably engage or attach to a bone anchor. A second handle is hingedly attached to the first handle and has a cannula attached thereto. The cannula has a central bore extending therethrough The cannula is aligned with the inserter shaft such that the inserter shaft is inside the central bore of the cannula and is extendible from and retractable in the cannula. Preferably, a biasing member is disposed between the first handle and the second and biases the first handle and the second handle apart.

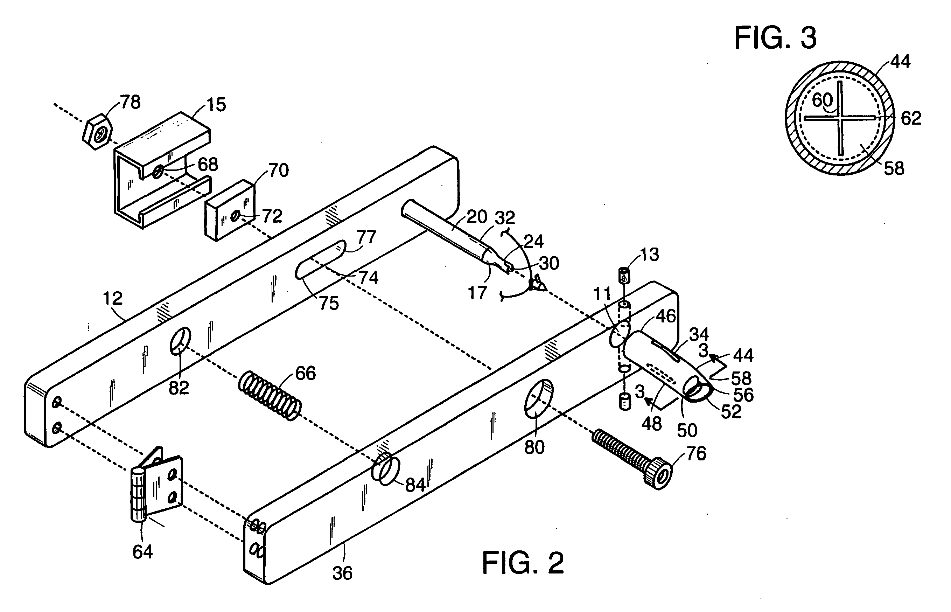

Figures 1 and 2 provide a plan view and an exploded view of an anchor implantation device 10 for introducing a bone anchor 22 transvaginally and driving it into the pubic bone or symphysis pubis. The device comprises a first handle 12 having a proximal end 14, a central region 16, and a distal end 18. The first handle 12 may be made of any relatively firm material, including plastic or metal Preferably, the first handle 12 is made of plastic, aluminum, stainless steel, or titanium. However, those skilled in the art will appreciate that a wide range of other material may also be employed.

The first handle 12 may be configured in any of a variety of shapes compatible with vaginal insertion. Preferably, the first handle 12 is rectangular. However, those skilled in the art will appreciate that a variety of configurations may be employed, such as a handle which tapers towards the distal end, and the present invention contemplates the use of any handle configuration compatible with vaginal insertion.

The dimensions of the first handle 12 are also compatible with vaginal insertion. The first handle 12 may be from about 10.16 cm (4 inches) to about 20.32 cm (8 inches) in length, about 0.64 cm (0.25 inches) to about 8.17 cm (1.25 inches) in width, and about 0.127 cm (0.05 inches) to about 1.27 cm (0.5 inches) in height. Preferably, the first handle 12 is about 12.7 cm (5 inches) to about 17.78 cm (7 inches) in length, about 1.27 cm (0.5 inches) to about 2.54 cm (1 inch) in width, and about 0.25 cm (0.1 inches) to about 0.762 cm (0.3 inches) in height. More preferably, the first handle 12 is has a length of 15.24 cm (6 inches), a width of 1.905 cm (0.75 inches) and a height of 0.508 cm (0.2 inches).

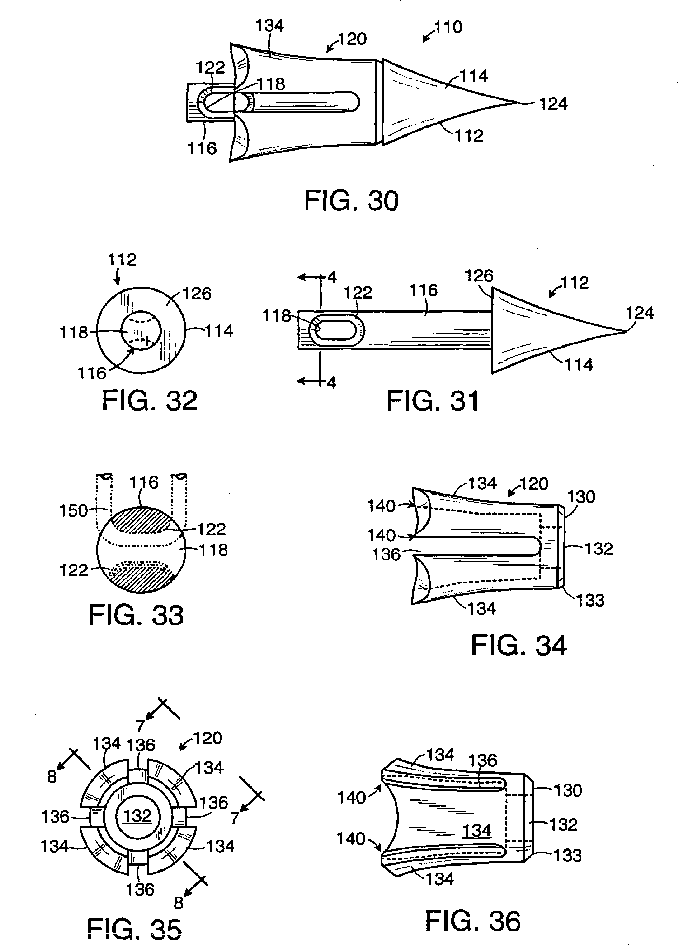

An inserter shaft 20 adapted for releasably engaging a bone anchor 22 is located near the distal end 18 of the first handle 12. A variety of bone anchors 22 can be used. In a preferred embodiment, illustrated in Figure 30 the bone anchor comprises a spear member 112 which is able to pierce and securely engage the bone. The spear member 112 has a generally cone shaped head portion 114 which is used to pierce the bone to which the and a shaft portion 116 with an oval eyelet 118 therethrough for receiving and holding a suture strand(s). To provide means for retaining the spear member 112 within the bone, the bone anchor 122 further comprises a collar member 120. The collar member is used for retaining the bone anchor 122 in place, once it has been driven into the bone, by lodging within the bone in a manner to resist removal of the bone anchor 122. The bone anchor 122 and its component parts are more fully described below.

The spear member 112 of the bone anchor 122 will now be described with additional reference to Figures 31-33. The shaft portion 116 of the spear member 112 is generally cylindrical in shape and has the eyelet 118, or bore, formed radially therethrough proximate one of its ends. The eyelet 118 may be oval, round or other suitable shapes and is of a sufficient size to permit suture strand or strands to pass therethrough. The circumference of each outer end of the eyelet 118 is chamfered or grounded to provide a bevel portion 122. It should be appreciated that the bevel portion 122 provides a generally smooth surface for contacting suture strand which has been passed through the eyelet 118. The eyelet 118 is located on the shaft portion 116 of the spear member 112 such that the transverse axis of the eyelet 118 intersect the longitudinal axis of the spear member 112.

The generally cone-shaped head portion 114 of the spear member 112 is located at an end of the shaft portion 116 opposite the end having the eyelet 118. As best shown in Figures 30 and 31, the apex of the cone-shaped head portion is a point 124 which is suitable for piercing and being driven into bone. The diameter of the cone-shaped head portion 114 increases, when viewed along a longitudinal direction rearwardly from the point 124 towards the shaft portion 116. The cone angle along this region is preferably about 30 degrees. The diameter of the cone-shaped head portion 114 increases at a greater rate along approximately the rearward half thereof, when viewed along the same longitudinal direction. Thus, the rearward half of the cone-shaped head portion 114 arcs outwardly from the central longitudinal axis of the spear member 112. As show in Figures 31 and 32, the base 126 of the cone-shaped head portion 114 is a ring-shaped planar surface which is oriented substantially perpendicular to the longitudinal axis of the shaft portion 116.

Preferably, the cone-shaped head portion 114 is formed integrally with the shaft portion 116 of the spear member 112. Alternatively, the cone-shaped head portion 114 and the shaft portion 116 may initially be formed separately and then subsequently attached to one another by any suitable means.

The collar member 120 of the bone anchor 122 will now be described with particular reference to Figure 30 and Figures 34-37. The collar member 120 is provided with a ring-shaped generally planar forward surface 130 which is adapted to bear against, and mate with the base 126 of the cone-shaped head portion 114 of the spear member 112. A circular bore 132 is located centrally through the planar forward surface 130 and is adapted to receive the shaft portion 116 of the spear member 112 therethrough. The circumference of the planar forward surface 130 may be, but is not necessarily, chamfered to formed a beveled outer rim portion 133.

Four separate flanges 134 extend rearwardly from the planar forward surface 130 of the collar member 120 as shown in Figures 34-39. The flanges 134 are separated from one another by longitudinally extending slots 136. The portions of the flanges 134 which are proximate to the planar forward surface 130 run generally parallel to the central longitudinal axis of the collar member 120. Each of the flanges 134 arcs generally outward from the central longitudinal axis as the flange 134 extends in a direction away from the planar forward surface 130. The lateral width of each of the flanges 134 increases as the flange 134 extends in a direction away from the planar forward surface 130. The extreme rearward end of each of the flanges 134 curves away from the planar forward surface 130 in the form a shallow C-shape, thereby providing two trailing tips 140 for each flange 134.

As set forth above, the collar member 120 is rotatably fitted over the shaft portion 116 of the spear member 112 to form the assembled bone anchor 122 as shown in Figure 1. While there is no need to permanently secure the collar member 120 to the spear member 112, the planar forward surface 130 may nevertheless be securely attached to the base 126 of the cone-shaped head member 114 of the spear member 112 by any suitable means. It will be appreciated, however, that by permitting the spear member 112 to freely rotate with respect to collar member 120, the suture strand 150 can be rotated by the surgeon after implantation to a position where the forces acting on the suture strand 150 by the bone anchor 122 are more evenly distributed around the region of the shaft portion 116 adjacent to the eyelet 118. Such a position of the suture strand 150 is shown in Figure 33.

In addition, it should also be appreciated that the two-piece construction of the bone anchor 122, affords machining advantages over a single-piece bone anchor. That is, it is easier to machine each of these components separately and to subsequently assemble them together, as opposed to machining the same basic structural features from a single piece of material. Any known materials suitable for orthopedic anchor devices may be employed to construct the bone anchor 122 of the present invention. Preferably, the bone anchor 122 is formed from a metallic material possessing sufficient strength to penetrate the bone. Such materials include titanium 316 LVM stainless steel, CoCrMo alloy, Nitinol alloy, or other suitable materials, Preferably, the bone anchor is made of titanium.

Referring now back to Figures 1 and 2, the inserter shaft 20 has a distal end 24, a central region 26, and a proximal end 28. Preferably, the inserter shaft extends at an angle of about 90° from the first handle 12.

The inserter shaft 20 may be made of any of a variety of materials, including steel, stainless steel, aluminum, titanium, and plastic, but is preferably made of stainless steel. Additionally, the inserter shaft 20 may have a variety of cross sectional shapes including rectangular, hexagonal, or triangular but preferably the inserter shaft 20 has a circular cross section.

The inserter shaft 20 maybe located from about 0.127 cm (0.05 inches) to about 1.27 cm (0.5 inches) from the distal end 18 of the first handle 12. preferably, the inserter shaft 20 is located from about 0.25 cm (0.1 inches) to about 0.762 cm (0.3 inches) from the distal end 18. More preferably, the inserter shaft 20 is located 0.508 cm (0.2 inches) from the distal end 18 of the handle.

The length of the inserter shaft 20 is consistent with transvaginal delivery of the releasable bone anchor 22. Thus, the inserter shaft 20 maybe from about 1.27 cm (0.5 inches) to about 3.81 cm (1.5 inches) long. Preferably, the inserter shaft 20 is from about 1.905 cm (0.75 inches) to about 3.175 cm (1.25 inches) in length. More preferably, the inserter shaft 20 is 2.54 cm (1 inch) in length.

Preferably, the proximal end 28 and the central region 26 of the inserter shaft 20 have an equal cross sectional area, which is larger than the cross sectional area of the bone anchor 22 and distal end 24. Thus, a shoulder 17 is formed at the junction between the central region 25 of the inserter shaft and the distal region of the inserter shaft. The shoulder 17 acts as a stop which will not penetrate the cortical shell of the bone.

The diameter of the inserter shaft is dependent upon the size of the bone anchor. In embodiments in which the inserter shaft 20 is cylindrical, the diameter of the proximal end 28 and central region 26 of the inserter shaft is from about 0.25 cm (0.1 inches) to about 0.762 cm (0.3 inches) and that of the distal end 24 is from about 0.102 cm (0.04 inches) to about 0.508 cm (0.2 inches.) Preferably, the diameter of the proximal end 28 and central region 26 of the inserter shaft is from about 0.381 cm (0.15 inches) to about 0.635 cm (0.25 inches) and that of the distal end 24 is from about 0.1778 cm (0.07 inches) to about 0.279 cm (0.11 inches). More preferably, the diameter of the proximal end 28 and central region 26 of the inserter shaft is 0.508 cm (0.2 inches) and that of the distal end 24 is 0.229 cm (0.09 inches).

Preferably, the inserter shaft 20 is curved as shown in Figures 1 and 2. As will be appreciated by those of skill in the art, the inserter shaft 20 may also be straight. In those embodiments in which the inserter shaft 20 is curved, the radius of curvature of the inserter shaft 20 is the distance between the pivot point of the hinge and the center of the inserter shaft. The radius of curvature of the inserter shaft 20 may be from about 8.89 cm (3.5 inches) to about 20.1 cm (7.9 inches) Preferably, the radius of curvature of the inserter shaft 20 is from about 11.94 cm (4.7 inches) to about 17.53 cm (6.9 inches). More preferably, the radius of curvature of the inserter shaft 20 is 14.73 cm (5.8 inches).

The distal end 24 of the inserter shaft 20 is adapted to releasably engage a bone anchor 22. In one embodiment, the bone anchor 22 is housed within a notch 30 at the distal end 24 of the inserter shaft, and frictionally engages the inner wall of the distal end 24 of the inserter shaft. However, it will be appreciated by those of skill in the art that the inserter shaft 20 may releasably engage the bone anchor 22 through a variety of means other than that described above, and such means are specifically contemplated by the present invention.

The distal end 24 of the inserter shaft maybe hollow or solid and has a complementary shape to the proximal end of the bone anchor 22 to permit the bone anchor 22 to frictionally engage the distal end 24 of the inserter shaft. For example, the distal end 24 of the inserter shaft and the proximal end of the bone anchor may be square, rectangular, pentagonal, triangular or hexagonal in cross section. Preferably, the distal end 24 of the inserter shaft and the proximal end of the bone anchor are cylindrical However, those skilled in the art will appreciate that numerous shapes may be employed, and the present invention specifically contemplates any such shape.

The central region 26 of the inserter shaft has a pair of grooves 32 therein for receiving a suture 54 attached to the bone anchor as illustrated in Figures I and 2. Preferably, the grooves 32 in the inserter shaft are coextensive with slots 34 in the outer cannula and are aligned with the slots 34.

The device also comprises a second handle 36 hingedly connected to the first handle 12 and having a proximal end 38, a central region 40, and a distal end 42. The second handle 36 may be fabricated from any of the materials discussed above with regard to the first handle 12. Additionally, the second handle 36 may have any of the dimensions and shapes discussed above with regard to the first handle 12. The preferred materials, dimensions, and shapes for the second handle 36 are the same as those discussed above with regard to the first handle 12.

The second handle 36 has a cannula 44 positioned near its distal end 42 and fixed within a bore 11 in the second handle by screws 13. The cannula 44 has a proximal end 46, a central region 48, and a distal end 50, with a central bore 52 running through its entire length. Preferably, the cannula 44 extends at an angle of about 90° from the second handle 36.

The cannula 44 may be fabricated from any of the materials described above with regard to the inserter shaft 20. Preferably, the cannula 44 is made of stainless steel.

The cannula 44 may have any of the shapes discussed above with regard to the inserter shaft 20. Preferably, the shape of the cannula 44 is the same as that of the inserter shaft 20.

The cannula 44 is located approximately the same distance from the distal end 42 of the second handle as the inserter shaft 20 is from the distal end 18 of the first handle and the central bore 52 of the cannula has an inner diameter larger than the outer diameter of the inserter shaft 20. In this way, the inserter shaft 20 extends into the central bore in the cannula as depicted in Figure 1. The inserter shaft 20 is extendible and retractable relative to the cannula 44.

Preferably, the cannula 44 has two oppositely disposed slots 34 therein through which the suture 54 attached to the bone anchor passes. These slots reduce the possibility of the suture 54 becoming tangled. Preferably, the slots 34 in the cannula are aligned with and coextensive with the grooves 32 in the inserter shaft.

Alternatively, the sutures can be contained within the cannula and extend out another portion of the device such as the first handle 12.

Preferably, the distal end 50 of the cannula has a sharp tip 56 to facilitate its use in piercing tissue.

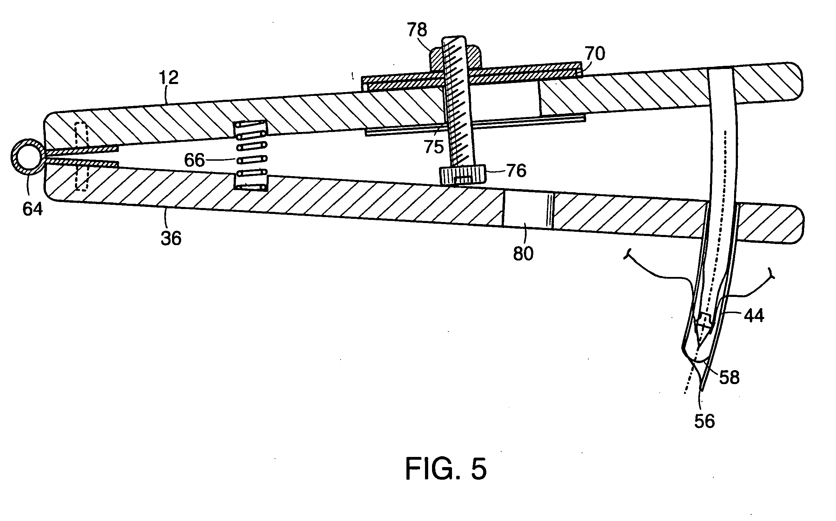

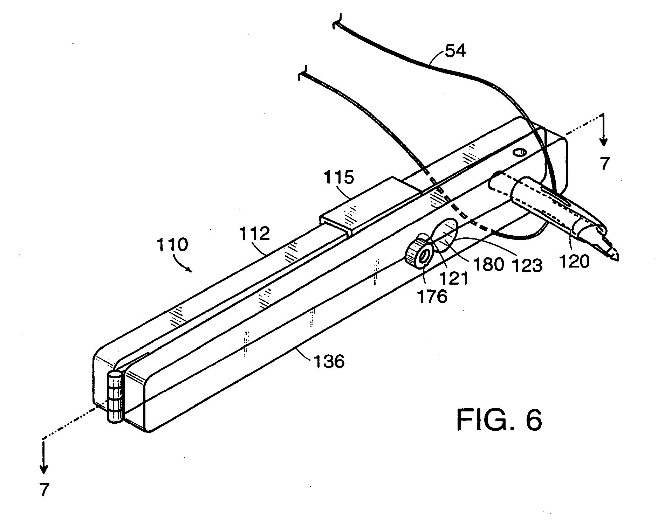

As illustrated in Figures 1 and 2, in the embodiments in which the inserter shaft 20 is curved, the cannula 44 is preferably also curved in the same arc as the inserter shaft 20. By curving the cannula 44, the diameter of the cannula 44 can be reduced in comparison with embodiments in which the inserter shaft and the cannula are not curved. Thus, in the embodiments in which the inserter shaft 20 and cannula 44 are curved, the inner diameter of the cannula 44 is from about 0.25 cm (0.1 inches) to about 0.64 cm (0.25 inches) and the outer diameter of the cannula 44 is from about 0.356 cm (0.14 inches) to about 0.788 cm (0.31 inches). Preferably the inner diameter of the cannula 44 is from about 0.381 cm (0.15 inches) to about 0.508 cm (0.2 inches). In a highly preferred embodiment, the inner diameter of the cannula is 0.432 cm (0.170 inches), the wall is about 0.051 cm (0.02 inches), and the outer diameter is about 0.533 cm (0.210 inches). These dimensions also apply to the devices in Figures 5 and 6 in which the inserter shaft and the cannula are also curved.

In a preferred embodiment, the cannula 44 has a protective cap 58 inside the central bore 52 and located at the distal end 50 of the cannula, as shown in Figures 2, 3 and 5. The protective cap 58 may be made of a variety of materials, such as plastic, thermoplastic elastomers, PET, PETG, rubber material, vinyl, gelatin, latex, thermoset rubbers and silicone. Preferably, the protective cap 58 is made of silicone or plastic.

The internal protective cap 58 acts to shield the bone anchor 22 from contamination, e.g., from contact with microorganisms in the vagina which could cause infection if introduced into the pubic bone during implantation of the bone anchor. In one embodiment, the protective cap 58 has a vertical slit 60 and a horizontal slit 62 therein which intersect to form a cross. Alternatively, the protective cap 58 has slits three slits which intersect to form a Y.

In one embodiment, the slits 60 and 62 penetrate entirely through the material of the protective cap 58, thereby dividing the protective cap into discrete segments. Preferably, the slits 60 and 62 are scored in the material of the protective cap 58 but do not extend entirely therethrough.

The slits 60 and 62 permit the bone anchor 22 to move through the protective cap 58 during implantation. In embodiments in which the slits 60 and 62 penetrate entirely through the material of the protective cap 58, the bone anchor 22 forces the segments of the protective cap 58 to separate as the bone anchor 22 is extended through the protective cap 58. The protective cap 58 remains in contact with the external surface of the bone anchor 22 as it is inserted into the bone, thereby shielding the bone anchor from contact with potentially infectious microorganisms in the vaginal wall.

The operation of the protective cap 58 in embodiments in which the slits 60 and 62 are scored in the protective cap is identical to that described above. However, in such embodiments, the tip of the bone anchor 22 pierces the material of the protective cap 58 as the bone anchor 22 is extended, thereby causing the protective cap 58 to separate into segments along the scores.

The proximal ends of the first and second handles, 14 and 38 respectively, are hingedly connected to permit them to move towards and away from one another. Any suitable type of hinge can be used, for example, this can be accomplished using a hinge 64 similar to that commonly found on doors as shown in Figures 1 and 2. Alternatively, a piece of rubber may be interposed between the first and second handles at their proximal ends 14 and 38 and secured thereto by bolts extending into holes in each of the handles. Those skilled in the art will appreciate that other means of hingedly connecting the first and second handles may be employed, and the present invention specifically contemplates embodiments in which such other hinging mechanisms are employed.

The first handle 12 and the second handle 36 are biased apart. In one embodiment, the biasing force is provided by a spring 66, as discussed below. The spring 66 can be metal, resilient polymer, pneumatically driven, or of any other suitable design. However, those skilled in the art will appreciate that a number of other structures can be employed to achieve the same biasing effect. The present invention specifically contemplates such other means of biasing the handles apart.

When sufficient force is applied to the distal ends of the first and second handles (18 and 42) to overcome the resistance of the spring, the distal ends (18 and 42) of the first and second handles move closer together. In the position in which the distal ends of the first and second handles are maximally separated, the inserter shaft 20 and bone anchor 22 thereon are fully retracted inside the cannula 44. As increasing force is applied to the handles and the distal ends approach one another, the inserter shaft 20 and bone anchor 22 thereon emerge from the distal end 50 of the cannula. At the point where the distal ends (18 and 42) of the first and second handles are touching, the inserter shaft 20 and bone anchor 22 thereon are maximally extended from the distal end 50 of the cannula.

At the point of maximum extension, the length of the inserter shaft 20 extending from the cannula 44 is from about 0.127 cm (0.05 inches) to about 2.032 cm (0.8 inches). Preferably, at the position of maximum extension, the length of the inserter shaft 20 extending from the cannula 44 is about 0.25 cm (0.1 inches) to about 1.27 cm (0.5 inches). More preferably, the length of the inserter shaft 20 extending from the cannula 44 at the position of maximum extension is 0.508 cm (0.2 inches).

In the above embodiment, the bone anchor 22 is inserted in the bone by manually moving the inserter shaft 20 axially through the bore 52 in the cannula until the inserter shaft and bone anchor 22 thereon extend from the cannula 44. However, the those skilled in the art will appreciate that approaches other than manually moving the inserter shaft may also be used to implant the bone anchor into the bone. For example, the bone anchor 22 can be forced into the bone by applying sufficient pneumatic pressure through the inserter shaft to eject the bone anchor from the inserter shaft with sufficient force to implant the bone anchor in the bone. Alternatively, the bone anchor may be driven into the bone by a spring mechanism.

Preferably, the device further comprises a locking mechanism for locking the device in the position in which the inserter shaft is fully retracted within the cannula in order to avoid accidental insertion of the bone anchor into tissue. As those skilled in the art will appreciate, a variety of locking structures may be used to achieve such locking.

One exemplary locking mechanism is shown in Figure 2. The locking mechanism comprises a locking plate 15 slidably mounted over the first handle 12 and having a bore 68 therein. The locking plate 15 is separated from the first handle 12 by a spacer 70 having an internally threaded bore 72 therein which is aligned with the bore 68 in the locking plate. The first handle 12 has an elongate hole 74 herein having a proximal end 75 and a distal end 77. A locking screw 76 extends through the elongate hole 74 in the first handle and the bores 72.68 in the spacer 70 and locking plate 15. The locking screw 76 is secured to the locking plate 15 by a nut 78.

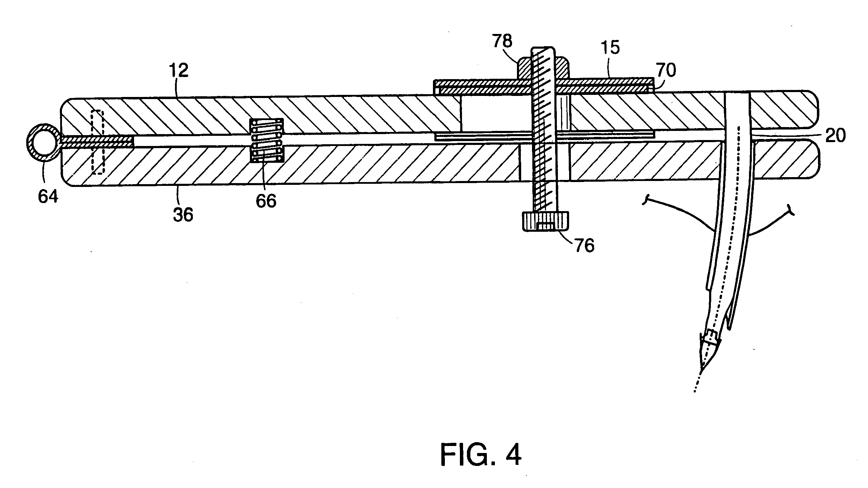

The second handle 36 has a bore 80 therethrough having a diameter larger than that of the head of the locking screw 76. In the unlocked position, the bore 80 can be aligned with the locking screw 76 as shown in Figure 4 thereby permitting the first handle 12 and the second handle 36 to be squeezed together such that the inserter shaft 20 extends from the cannula 44.

As illustrated in Figure 5, in the locked position, the locking plate 15 is positioned at the proximal end 75 of the elongate hold 74 in the first handle. In this position, the locking screw 76 is disposed between the first and second handles such that the head of the screw abuts the inner side of the second handle 36, thereby preventing the first handle 12 and the second handle 36 form being squeezed together.

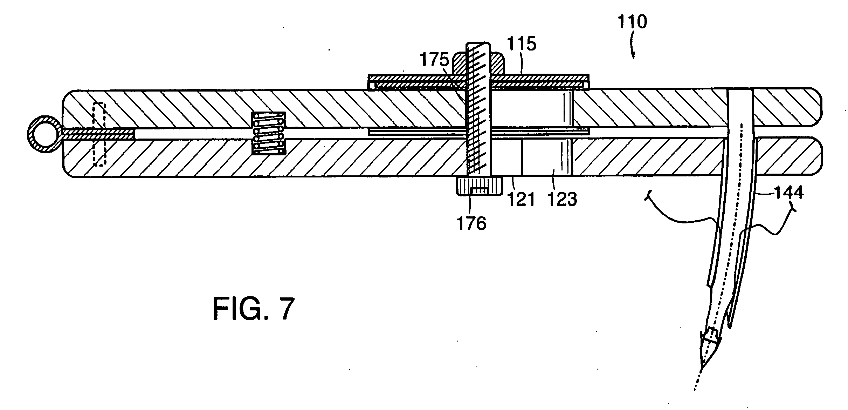

In an alternate embodiment, the bone anchor implantation device 110 may have a dual position lock permitting the device to be locked in a position in which the inserter shaft 120 is fully retracted within the cannula 144 or in a position in which the inserter shaft 120 is fully extended from the cannula 144. In this embodiment, illustrated in Figure 6 the bore 180 of the second handle 136 is keyhole shaped. The narrower part 121 of the keyhole shaped bore is sufficiently narrow to prevent the head of the locking screw 176 from passing therethrough. As illustrated in Figure 7, when the locking plate 115 is positioned at the proximal end 175 of the elongate bore in the first handle, the head of the locking screw 176 is over the narrow part 121 of the keyhole shaped aperture 180. As shown in Figure 7, in this position the inner side of the head of locking screw 176 contacts the outer side of the second handle. The device is locked in the position in which the inserter shaft and bone anchor thereon are fully extended.

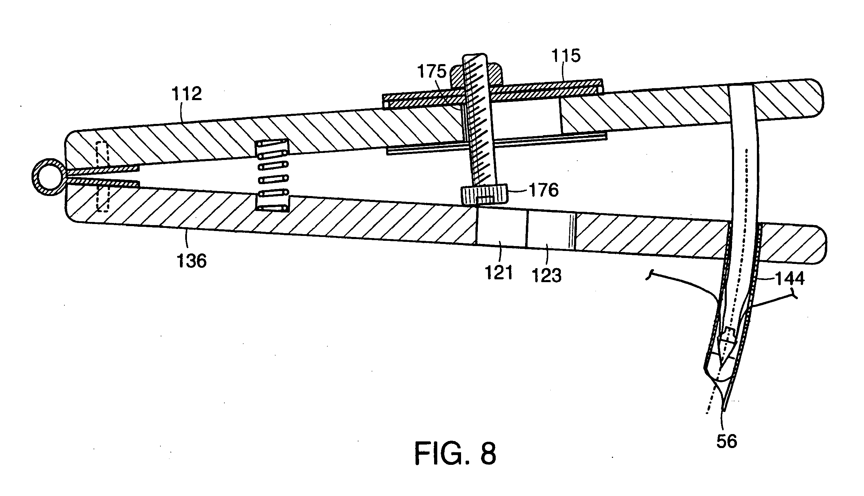

When the locking plate 115 is positioned at the distal end 177 of the elongate bore in the first handle, the head of locking screw 176 is aligned with the wide portion 123 of the keyhole shaped bore. The locking plate 115 can then be returned to the proximal end 175 of the elongate hole, such that the head of the locking screw 176 is disposed between the first handle 112 and the second handle 136 and the head of the locking screw 176 abuts the inner side of the second handle 136, as shown in Figure 8. In this position the inserter shaft 120 is fully retracted and the first handle 112 and the second handle 136 cannot be squeezed together.

The above locking mechanisms may be used in the embodiments where the inserter shaft and cannula are straight. As those skilled in the art will appreciate, a variety of other locking structures may be used to achieve such dual position locking. Such other locking mechanisms are specifically contemplated by the present invention.

In the embodiments described above, the force biasing the two handles apart is preferably provided by a spring 66 disposed between two depressions 82 and 84 in the first handle 12 and the second handle 36. In the embodiments described above, the spring 66 is located in the central regions 16 and 40 of the first and second handles. However, those skilled in the art will appreciate that the location of the spring is not critical to the operation of the present invention. Additionally, it will be appreciated that biasing members other than a spring may be employed to bias the handles apart.

Using the present bone anchor implantation device, the bone anchor is transvaginally introduced into the pubic bone as follows.

After making an incision in the anterior vaginal wall, the endopelvic fascia is accessed using techniques well known to those of skill in the art, such as with a conventional retractor. A Foley catheter may be introduced to assist in locating the bladder neck. The bone anchor implantation device is inserted into the vaginal introitus and the first desired site for bone anchor implantation is located by digital palpation of the urethra, pubic symphysis or other anatomical landmark or other techniques known to those of ordinary skill in the art. The device is locked in the position in which the inserter shaft is fully retracted during this procedure.

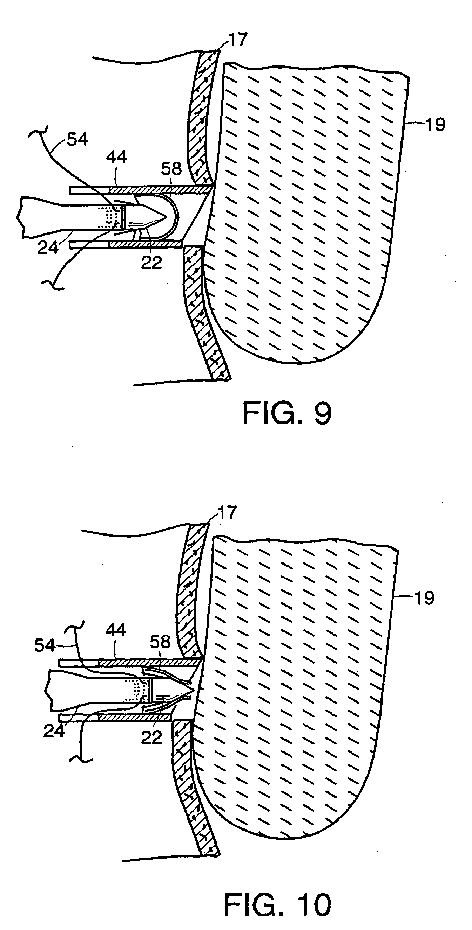

Once the desired site for bone anchor implantation is located, the sharp tip 56 on the distal end of the cannula is driven through the endopelvic fascia 17. The pointed end of the cannula can also be employed to locate the desired implantation site by inserting the device into the vaginal introitus and through the incision, piercing the endopelvic fascia, and moving the cannula along the pubic bone 19 to the desired implantation site, as shown in Figure 9.

The device is then unlocked from the position in which the inserter shaft 20 is fully retracted. In the embodiment having a single position lock, the first and second handles (12 and 36) are pressed together with enough pressure to extend the inserter shaft 20 out of the cannula 44 and drive the bone anchor 22 into the posterior portion of the bone 19. Alternatively, in the embodiment having a dual position lock, the device is locked in the position in which the inserter shaft 120 is fully extended from the cannula 144 and the manual pressure is applied to drive the bone anchor 122 into the posterior portion of the pubic bone 19.

As shown in Figure 10, when the first and second handles are squeezed towards one another, the inserter shaft moves towards the bone 19. The bone anchor 22 pierces the protective cap 58 which separates as the bone anchor 22 passes therethrough. The protective cap 58 shields the bone anchor 22 from contact with the vaginal tissue.

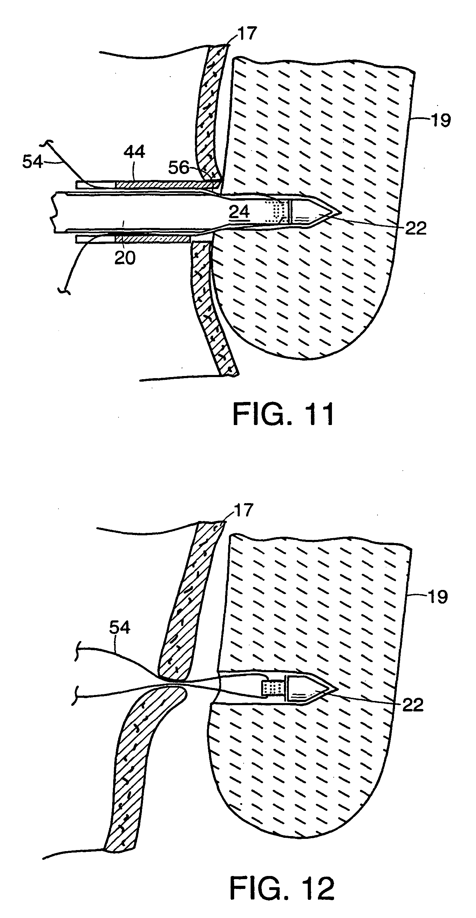

As shown in Figure 11, when the inserter shaft 20 is extended beyond the distal tip of the cannula 56, the bone anchor contacts the bone 19 and is driven therein.

The inserter shaft 20 is then retracted into the cannula 44, leaving the bone anchor 22 implanted in the bone 19 with the attached suture 54 extending through the wound in the vaginal wall and the endopelvic fascia as shown in Figure 12.

The above site location and bone anchor implantation procedure is repeated to implant a second bone anchor on the opposite side of the urethra from the first bone anchor.

In one embodiment, the sutures are attached to a needle, looped back through the vaginal wall, and attached to tissue such as the endopelvic fascia or the vaginal wall so as to bias the tissue surrounding the urethra towards the urethra. The biasing force compresses or stabilizes the bladder neck thereby maintaining or improving urinary continence.

Alternatively, the sutures attached to the bone anchors can be attached to a sling which compresses or stabilizes the bladder neck. In such procedures, an incision is made midline to the urethra. An opening or pocket for receiving the sling is created in the tissue between the urethra and the upper vaginal wall. The bone anchor implantation device is inserted through the incision, into the pocket, and through the endopelvic fascia to contact the pubic bone. At least one bone anchor is inserted into the pubic bone on each side of the urethra. The sling is introduced into the opening or pocket and attached to the sutures. The tension on the sling provided by the sutures is adjusted to provide the appropriate biasing force to the urethra.

Example 1 describes one method of using the present bone anchor implantation device to compress or stabilize the bladder neck with sutures. it will be appreciated that the bone anchor implantation device can be used with other methods in which sutures compress or stabilize the bladder neck.

### EXAMPLE 1

### Compression or Stabilization of the Bladder Neck with Sutures

The bone anchor implantation device can be used in incontinence treatments in which the bladder neck is compressed or stabilized with sutures. A Foley catheter is inserted into the urethra to indicate its location. An incision is then made through the anterior vaginal wall, preferably approximately 1 cm lateral to midline and adjacent to the bladder neck. The vaginal wall is retracted to allow access to the endopelvic fascia. The bone anchor implantation device, having a bone anchor with sutures attached thereto releasably engaged with the inserter shaft, is introduced through the opening in the vaginal wall with the device locked in the position in which the inserter shaft is fully retracted within the cannula, and the sharp point is pressed through the fascia to contact,the posterior pubic bone. Preferably, the anchor implantation site is located lateral to the symphysis pubis and cephalad to the inferior edge of the pubic bone. The anchor implantation site is located by palpating the inferior rim of the pubic bone and the symphysis pubis, moving laterally until the lower border of the obturator foramen is located. Preferably, the anchor is located from about 0.5 to 4 cm lateral to the symphysis pubis and from about 0.5 to 3 cm cephalad to the inferior edge. More preferably, the anchor implantation site is located approximately 1 cm lateral to the symphysis pubis and 1 cm cephalad to the inferior edge of the pubic bone. In addition, the anchor implantation site can be located on the pubic ramus.

The locking mechanism of the bone anchor implantation device is then placed in the unlocked position, and the two handles are squeezed together such that the inserter shaft is in the extended position. Alternatively, for devices having a dual position locking mechanism, the bone anchor may be exposed by locking the device in the position in which the inserter shaft is fully extended from the cannula. In either case, the anchor is driven into the pubic bone using manual pressure and opposing thumb pressure on the external pubic section if necessary.

The bone anchor implantation device is withdrawn, leaving the two free ends of the anchored suture exiting the endopelvic fascia 17. A device such as a Mayo needle is then attached to one free end of the anchored suture and a "bite of fascia" is taken adjacent to the bladder neck. Preferably, the entry and exit points of the suture are adjacent to the bladder neck approximately 0.5 cm lateral to the urethra. This step is then repeated with the other free end of the suture, and the two ends are tied together. The vaginal wall incision is then closed.

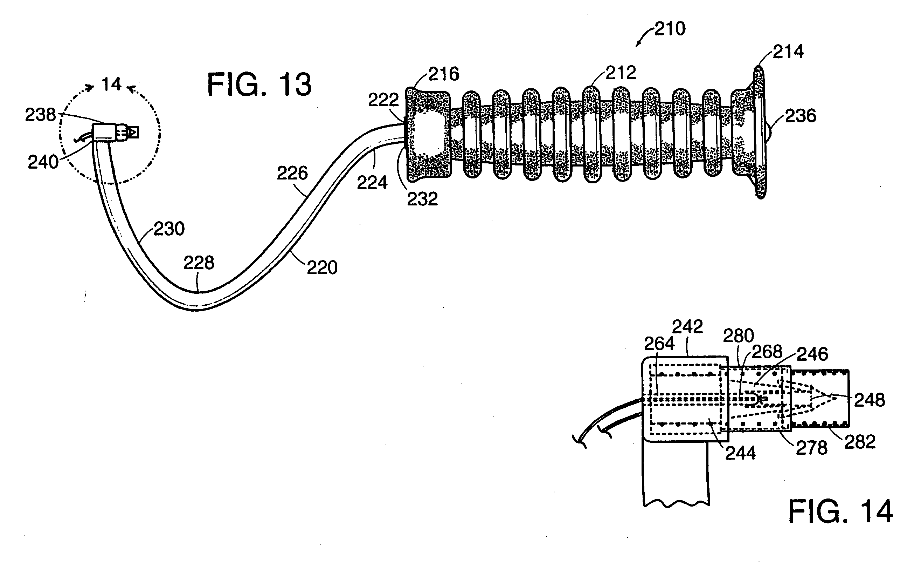

Alternatively, the entry and exit points of the suture can be made as illustrated in Figure 13a of U.S. Patent No. 5,611,515 to Benderev.

The above procedure is then repeated on the opposite side of the urethra to complete the bladder neck suspension. The sutures are then appropriately tensioned. Appropriate tension is confirmed using well known means such as cystoscopy or a standard Q tip test.

### EXAMPLE 2

Example 2 describes use of the bone anchor implantation device in a procedure in which the bladder neck is compressed or stabilized with a sling. However, it will be appreciated that the bone anchor implantation device can be used with other methods in which the bladder neck is compressed or stabilized with a sling.

### Double Anchor Placement: For Sling Or Bolster Procedure

The bone anchor implantation device can also be used in incontinence treatments in which the bladder neck is compressed or stabilized using a sling. Preferably, in such procedures two bone anchors are placed on either side of the urethra. However, one of ordinary skill in the art will appreciate that one or more than two bone anchors per side can be used. The procedure is performed as follows.

A Foley catheter is inserted into the urethra to indicate its location. Starting adjacent to the bladder neck on either side of the urethra, a 1 cm incision is made through the anterior vaginal wall approximately 1 cm lateral to and parallel to the midline of the urethra. The vaginal wall is retracted to allow access to the endopelvic fascia 17. Blunt dissection is used to tunnel under the urethra and form a pocket for the sling.

The bone anchor implantation device is introduced through the opening in the vaginal wall with the device locked in position in which the inserter shaft is fully retracted within the cannula, and the sharp point of the cannula is pressed through the fascia 17 near the distal end of the vaginal wall incision closer to the bladder neck, to contact the posterior aspect of the pubic bone. Preferably, the first anchor implant site is located lateral to the symphysis pubis and cephalad to the inferior edge of the pubic bone. More preferably, the first anchor implant site is located approximately 1 cm lateral to the symphysis pubis and 1 cm cephalad to the inferior edge of the pubic bone.

The locking mechanism of the bone anchor implantation device is then placed in the unlocked position, and the two handles are squeezed together to expose the anchor. Alternatively, for devices having a dual position locking mechanism, the bone anchor may be exposed by locking the device in the position in which the inserter shaft is fully extended from the cannula. The anchor is driven into the pubic bone using manual pressure and opposing thumb pressure on the external pubic region if necessary.

The bone anchor implantation device is withdrawn leaving the two free ends of suture exiting the endopelvic fascia.

The above bone anchor implantation procedure is repeated to introduce a second anchor on the same side of the urethra as the first anchor. The second anchor implant site is located by palpating the obturator foramen in the pelvis just cephalad to the ramus. For implantation of the second anchor, the fascial tissue near the proximal end of the vaginal wall incision farther from the bladder neck is pierced. The second anchor is implanted on the superior (cephalad) aspect of the ramus.

The bone anchor implantation device is removed as before trailing the two free ends of each suture from the vaginal wall incision.

The above procedures for implantation of the first and second anchors are repeated on the opposite side of the urethra.

The sling is then positioned in the pocket under the urethra. The free ends of suture from the two anchors on each side of the urethra are then tied to the corresponding corners of the sling. The sutures are then tied off with the appropriate amount of tension to suspend or stabilize the bladder neck. The vaginal wall incisions are then closed on each side.

Alternatively, the above procedure can also be utilized in techniques in which only a single bone anchor is inserted on either side of the urethra. Preferably, in such procedures the anchor implantation site is located approximately 1 cm lateral to the symphysis pubis and 1 cm cephalad to the inferior edge of the pubic bone. More preferably, the anchor implantation site is located approximately 1 cm lateral to the symphysis pubis and 1 cm cephalad to the inferior edge of the pubic bone.

### Bone Anchor Implantation Device With Hooked Shaft

In another embodiment, the anchor implantation device of the present invention has a hooked shaft with a bone anchor mount for releasably engaging a bone anchor on the distal end of the shaft. This embodiment reduces the amount of force required to drive the bone anchor into the bone by utilizing the patient's body weight to provide an opposing force.

In this embodiment, the anchor implantation device comprises a handle, a hooked shaft secured to the handle and a bone anchor mount adapted to releasably engage a bone anchor attached to the distal end of the shaft. The bone anchor mount generally points toward the handle, such that the user can drive the bone anchor into the bone by simply pulling back on the handle and using the patient's body weight to provide an opposing force. Preferably, the longitudinal axis of the bone anchor mount is aligned with the longitudinal axis of the handle. Preferably, a protective sheath is attached to the bone anchor mount such that the bone anchor releasably engaged to the bone anchor mount is enclosed within the protective sheath and isolated from tissue contact during placement of the device. More preferably, the protective sheath is a telescoping sheath or a balloon.

A representative anchor implantation device having a hooked shaft is shown in Figure 13. As illustrated in Figure 13, the anchor implantation device 210 has a handle 212 having a proximal end 214 and a distal end 216. The handle 212 may be made of a variety of materials, such as plastic or metal.

The shaft 220 may be made of a variety of materials such as stainless steel engineering plastics, fiber-bearing components, or other materials. Preferably, the shaft is made of stainless steel.

In the embodiment of the bone anchor implantation device shown in Figure 13, shaft 220 comprises a straight proximal section 222, a first generally curved section 224 distal to the straight proximal section, a second generally curved section 226 distal to the first curved section, a third generally curved section 228 distal to the second curved section, and a fourth generally curved section 230 distal to the third curved section. However, one skill in the art would appreciate that the shaft could also comprise a series of straight segments angled relative to one another to form a hook.

The straight proximal section 222 of the shaft 220 has an annular shoulder 232 which abuts the distal end 216 of the handle. The straight proximal section 222 passes through a lumen (not shown) extending through the handle. The proximal end of the straight proximal section 222 has a threaded bore which is adapted to receive a screw 236 which secures the shaft 220 to the handle. If desired, a washer (not shown) may be placed between the proximal end 214 of the handle and the screw 236.

While one means of securing the shaft 220 to the handle 212 was described above, those skilled in the art wilt appreciate that a variety of other means may be employed. For example, a plastic handle may be formed over the shaft such that the shaft is integral with the handle.

The straight proximal section 222 of the shaft 220 may be from about 7.62 cm (3 inches) to about 15.24 cm (6 inches) in length. Preferably, the straight proximal section 222 is from about 10.16 cm (4 inches) to about 12.7 cm (5 inches) in length. More preferably, the straight proximal section 222 is about 11.43 cm (4.5 inches) in length.

The handle 212 defines an axis at the proximal end of the anchor implantation device 210, and ten moving distally from the handle 212 the shaft 220 first curves away from the axis of the handle and then back toward the axis of the handle 212. The distal end of the shaft 220 preferably is located in the vicinity of the axis of the handle 212. In some preferred embodiments, the shaft 220 at the distal end can be generally perpendicular to the axis of the handle or can actually be curving back toward the handle 212. Preferably the distance from the distal end of the handle 212 to the tip of the tapered bone anchor receptacle 246 measured along the longitudinal axis of the handle 212 is about 8.57 cm (3_{3/8} inches). Preferably, the distance from the distal end of the handle 212 to the distal end of the bone anchor mount 238 is about 10.16 cm (4 inches). Preferably, the distance of a line perpendicular to the longitudinal axis of the handle 212 extending from the bottom of the third curved section 228 is about 5.08 cm (2 inches).

Referring to Figures 13-16, a bone anchor mount 238 is attached to the distal end 240 of the fourth curved section 230 of the shaft 220. The bone anchor mount 238 may be oriented at an angle from about 60° to about 120° relative to the distal end 240 of the fourth curved section. Preferably, the bone anchor mount 238 is oriented at an angle from about 80° to about 100° relative to the distal end 240 of the fourth curved section. More preferably, the bone anchor mount 238 is oriented at an angle of approximately 90° relative to the distal end 240 of the fourth curved section, as illustrated in Figure 13.

The bone anchor mount comprises an outer cylinder 242, an inner cylinder 244, and a tapered bone anchor receptacle 246 for releasably engaging a bone anchor 248. As was the case with the two handle bone anchor implantation device discussed above, a variety af bone anchors can also be used with the bone anchor implantation device having a hooked shaft. Preferably the bone anchor used with the hooked shaft device is the bone anchor discussed above with respect to the two handle bone anchor implantation device.

In any event, it is preferred that the bone anchor mount 238 and the bone anchor receptacle 246 are oriented so that the bone anchor 248 is pointed in the general direction of the handle 212. In one preferred embodiment, the axis of the bone anchor 248 is generally aligned with the axis of the handle 212, with the bone anchor pointed toward the handle 212.

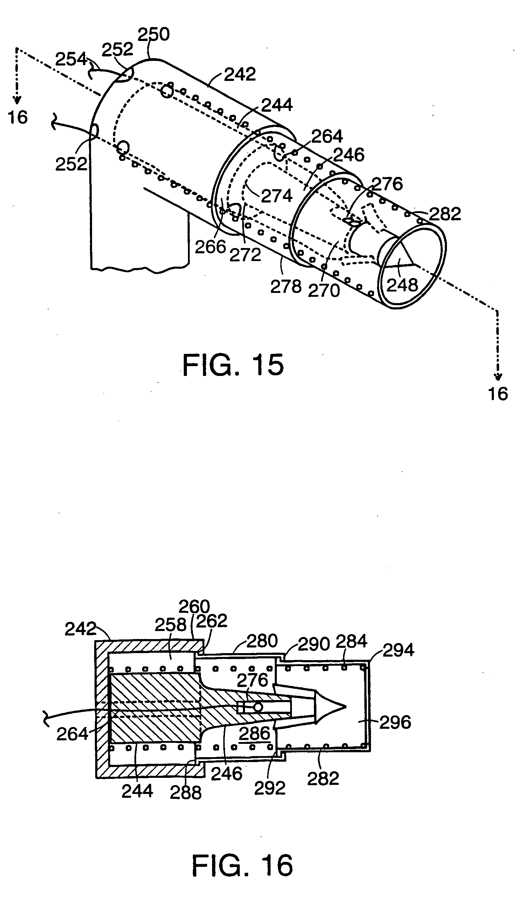

The bone anchor mount 23 8 may be fabricated from the same materials as the shaft 220 and may be attached to the shaft 220 by a variety of methods known to those skilled in the art, such as brazing. As best shown in Figure 15 the distal end 250 of the outer cylinder 242 has a pair of holes 252 therein sized to accommodate a suture 254.

The outer cylinder 242 may have a diameter from about 0.457 cm (0.18 inches) to about 1.524 cm (0.6 inches). Preferably, the outer cylinder 242 has a diameter from about 0.635 cm (0.25 inches) to about 1.27 cm (0.5 inches). More preferably, the outer cylinder 242 has a diameter of about 0.953 (0.375 inches).

As best shown in the cross section of Figure 16, the outer cylinder 242 has a cavity 258 formed therein, creating a cup in the proximal region of the outer cylinder 242. The proximal end 260 of the outer cylinder 242 has an annular shoulder 262 thereon.

The inner cylinder 244 is connected to the outer cylinder 242 and extends into the cavity 258 as best shown in Figure 16. The inner cylinder 244 may be connected to the outer cylinder 242 in a variety of ways known to those skilled in the art. For example, the inner cylinder 244 may be fused to the outer cylinder 242. As best shown in Figure 15, the inner cylinder 244 has grooves 264 therein adapted to accommodate the suture 254.

A tapered bone anchor receptacle 246 extends from the proximal end 266 of the inner cylinder 244. The tapered bone anchor receptacle 246 has grooves 268 therein adapted to accommodate the suture 254.

The tapered bone anchor receptacle 246 may extend from the proximal end 266 of the inner cylinder 244 by a distance of from about 0.762 cm (0.3 inches) to about 1.778 cm (0.7 inches). Preferably, the tapered bone anchor receptacle 246 extends from the proximal end 266 of the inner cylinder 244 by a distance of from about 1.016 cm (0.4 inches) to about 1.524 cm (0.6 inches). More preferably, the tapered bone anchor receptacle 246 extends from the proximal end 266 of the inner cylinder 244 by a distance of about 1.27 cm (0.5 inches).

The distal end 270 of the tapered bone anchor receptacle 246 preferably has a width smaller than that of the proximal end 266 of the inner cylinder 244. This configuration produces a shoulder 272 which may serve as a depth stop to ensure that the bone anchor 248 is driven into the bone to the desired depth.

The distal end 270 of the tapered bone anchor receptacle 246 may be from about 0.203 cm (0.08 inches) to about 0.305 (0.12 inches) in width. Preferably, the distal end 270 of the tapered bone anchor receptacle 246 is from about 0.229 cm (0.09 inches) to about 0.279 cm (0.110 inches) in width. More preferably, the distal end of the tapered bone anchor receptacle 246 is 0.254 cm (0.1 inches) in width.

The proximal end 274 of the tapered bone anchor receptacle 246 may be from about 0.279 cm (0.110 inches) to about 0.381 cm (0.15 inches) in width. Preferably, the proximal end 274 of the tapered bone anchor receptacle 246 is from about 0.305 cm (0.12 inches) to about 0.356 cm (0.14 inches) in width. More preferably, the proximal end 274 of the tapered bone anchor receptacle 246 is 0.33 cm (0.13 inches) in width.

The proximal end 274 of the tapered bone anchor receptacle 246 may have a variety of cross sectional shapes adapted to releasably engage the bone anchor 248. For example, the proximal end 274 of the tapered bone anchor receptacle 246 may be square, rectangular, pentagonal, triangular or hexagonal in cross section.

As depicted in Figures 14-16, the tapered bone anchor receptacle 246 may have a notch 276 therein in which the bone anchor 248 is releasably seated.

Alternatively, the outer cylinder, inner cylinder, and tapered bone anchor receptacle may be a single integral component.

Preferably, the bone anchor implantation device has a protective sheath connected to the bone anchor mount which protects the point of the bone anchor from tissue contact during placement of the device and also protects the bone anchor from contacting potentially infectious microorganisms.

One embodiment of the protective sheath 278 is shown in Figures 13-16. In this embodiment, the protective sheath 278 comprises a first telescoping cylinder 280 and a second telescoping cylinder 282. A spring 284 biases the first telescoping cylinder 280 and the second telescoping cylinder 282 to a position in which they extend from the outer cylinder 242 and cover the bone anchor 248.

The first and second telescoping cylinders 280, 282 may be made of a variety of materials such as stainless steel or plastic. Preferably, the first and second telescoping cylinders 280, 282 are made of stainless steel.

The first telescoping cylinder 280 has a lumen 286 extending therethrough. The first telescoping 280 cylinder has a first shoulder 288 which engages shoulder 262 on the outer cylinder 242 and a second shoulder 290 which engages a first shoulder 292 on the second telescoping cylinder 282.

The second telescoping cylinder 282 has a first shoulder 292 which engages the second shoulder 290 on the first telescoping cylinder 280 as described above. A second shoulder 294 is located at the proximal end of the second telescoping cylinder 282 and engages the spring 284. The second telescoping cylinder 282 also has a lumen 296 extending therethrough which is in fluid communication with the lumen 286 of the first telescoping cylinder 280 and the cavity 258 in the outer cylinder 242.

The inner diameter of the first telescoping cylinder 280 is slightly larger than the outer diameter of the second telescoping cylinder 282 such that the second telescoping cylinder 282 can retract inside the first telescoping cylinder 280. The first telescoping cylinder 280 and the second telescoping 282 can retract inside the cavity 258 of the outer cylinder 242.

The first telescoping cylinder 280 may be from about 0.508 cm (0.2 inches) to about 0.762 cm (0.3 inches) in length, with an inner diameter of from about 0.686 cm (0.27 inches) to about 0.838 cm (0.33 inches) and an outer diameter of about 0.762 cm (0.3 inches) to about 0.914 cm (0.36 inches). Preferably, the first telescoping cylinder 280 is from about 0.584 cm (0.23 inches) to about 0.686 cm (0.27 inches) in length, with an inner diameter of from about 0.737 cm (0.29 inches) to about 0.787 cm (0.31 inches) and an outer diameter of about 0.813 cm (0.32 inches) to about 0.864 cm (0.34 inches) More preferably, the first telescoping cylinder 280 is about 0.635 cm (0.25 inches) in length, with an inner diameter of about 0.762 cm (0.3 inches) and an outer diameter of about 0.838cm (0.33 inches).

The second telescoping cylinder 282 may be from about 0.508 cm (0.2 inches) to about 0.762 cm (0.3 inches) in length, with an inner diameter of from about 0.559 cm (0.22 inches) to about 0.787 cm (0.31 inches) and an outer diameter of about 0.635 cm (0.25 inches) to about 0.889cm (0.35 inches). Preferably, the second telescoping cylinder 282 is from about 0.584 cm (0.23 inches) to about 0.686 cm (0.27 inches) in length, with an inner diameter of from about 0.24 inches to about 0.737 cm (0.29 inches) and an outer diameter of about 0.686 cm (0.27 inches) to about 0.838 cm (0.33 inches). More preferably, the second telescoping cylinder 282 is about 0.25 inches in length, with an inner diameter of about 0.27 inches and an outer diameter of about 0.3 inches.

As illustrated in Figures 13-16, a spring 284 biases the first and second telescoping cylinders 280 and 282 towards a position in which the first telescoping cylinder 280 and the second telescoping cylinder 282 are extended from the outer cylinder 242.

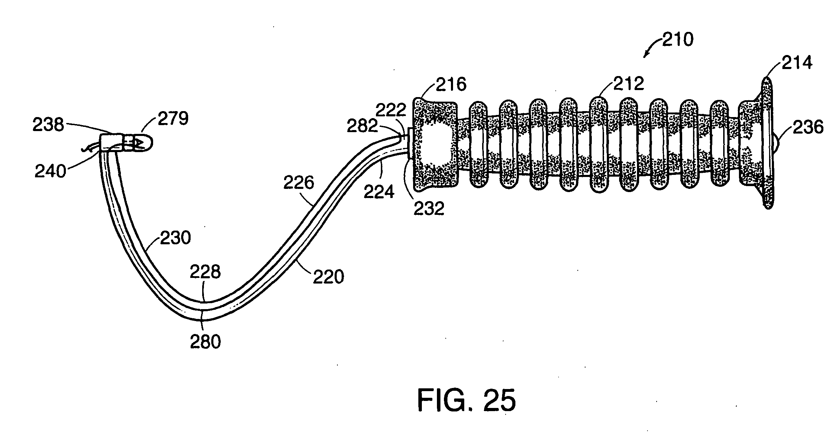

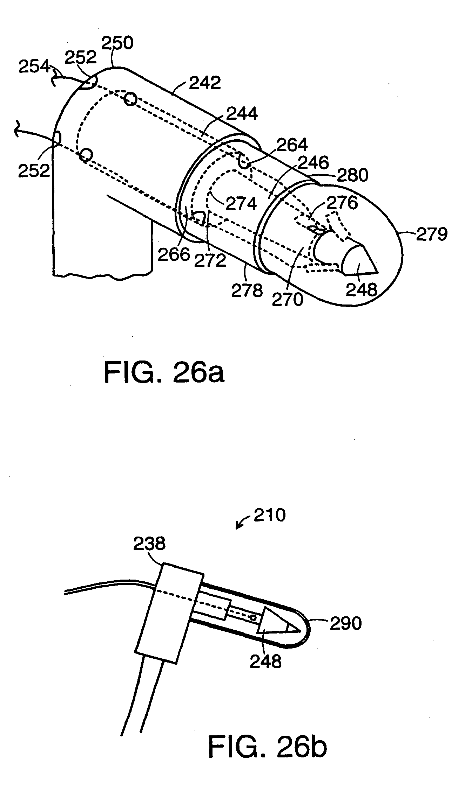

Another embodiment of the protective sheath is depicted in Figures 25 and 26a. The bone anchor implantation device as described above with respect to Figures 14-17 has a balloon which encapsulates the bone anchor. The bone anchor implantation device 210 has a balloon 279 which is coupled to the bone anchor mount 238 and which covers the bone anchor 248. The balloon 279 protects the bone anchor from contacting potentially infections microorganisms prior to implantation.

The balloon 279 may be made of any material which exhibits a strength that allows it to be punctured by the bone anchor. Examples of suitable materials include plastic, thermoplastic, elastromers, PET, PETG, rubber, vinyl, latex, gelatin or silicone. In a preferred embodiment, the balloon is made of latex.

Alternatively, the balloon 279 can be made of a biodegradable material. A suitable biodegradable material dissolves within the patient after a predetermined period of time. Following implantation of the bone anchor the punctured sheath remains are simply metabolized by natural biological processes.

In a preferred embodiment, the balloon comprises a biodegradable polymer. The polymer may be either natural or synthetic. Synthetic polymers offer greater advantages that natural materials in that they can be tailored to give a wider range of properties and are more uniform than materials from natural sources. Synthetic polymers also offer a more reliable source of raw materials which reduce the risks of invoking an immunogenic response.

Biodegradable polymers are synthesized from chemical functional groups such as, for example, esters, anhydrides, orthoesters and amides, which have hydrolytically unstable linkages in the backbone. Preferably, the balloon is made of a biodegradable materials such as polyglycotic acid (PGA), polylactic acid (PLA), poly (dioxanone) (PDO), poly (1-lactide) (LPLA), poly (dl-lactide) (DLPLA), poly (glycolide-co-trimethylene carbonate) (PGA-TMC), poly (1-lactide-co-glycolide) (PGA-LPLA), poly (dl-lactide-co-glycolide) (PGA-DLPLA), poly (1-lactide-co-dl-lactide) (LPLA-DLPLA), poly(glycolide-co-trimethylene carbonate-co-dioxanone) (PDO-PGA-TMC), poly(ε-caprolactone), poly(dioxanone)(a polyether-ester), poly (lactide-co-glycotide), poly(SA-HDA anhydride), poly(orthoester), polyglyconate.

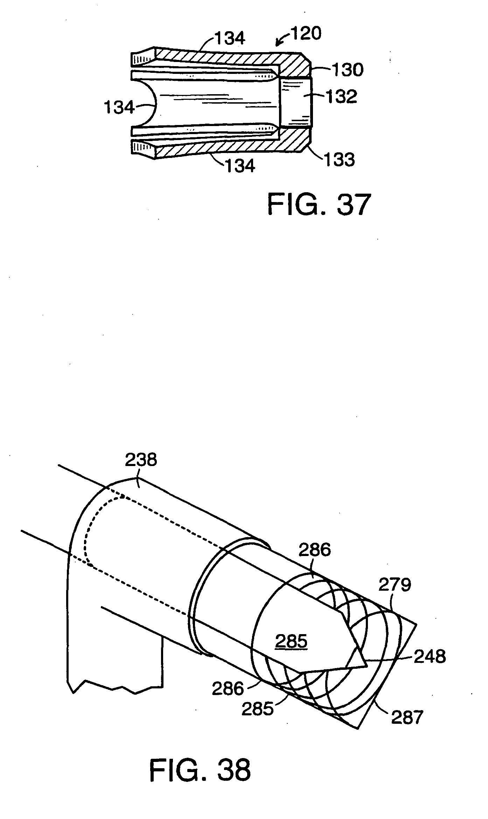

The balloon or structure can be filled with air, water, antibiotic or any substance which inflates the balloon so as to prevent the bone anchor from puncturing the balloon prior to implantation. Alternatively, as illustrated in Figure 38 and discussed below, the device may further include a spring which is attached to the shaft within the balloon to maintain the shape of the balloon and prevent the bone anchor from puncturing the balloon prematurely. In a preferred embodiment, the balloon 278 contains an antibiotic that is released when the sheath is perforated by the bone anchor during implantation. The balloon may contain several antibiotics that have complementary activity. The antibiotic prevents infection at the site where the bone anchor is pressed into the bone. Non-limiting examples of suitable antibiotics for use in the invention include nafcillin, aminogylcoside, ciprofloxin, clindamcin, piperacillin/tazobactum, ampicillin/sulbactum, aminoglcoside, vancomycin, cephalosporin, TMP/SMX, ampicillin, gentaminicin, tobramycin and ciprofloxacin.

There are numerous ways to insert an antibiotic or other desired substance into the balloon. In one embodiment, the bone anchor implantation device further comprises an port 280 which allows antibiotics to be inserted into the balloon. As shown in Figure 25, the port 280 extends from the balloon 279 into a lumen which extends from one end of the shaft to the other. The port 280 has an opening 282 at the distal end of the shaft. Antibiotics inserted into the opening 282 travel through the port 280 into the balloon 279. In an alternate embodiment (not shown), the port extends from the balloon into a lumen which extends from one end of the shaft to the other end and through the handle to an opening. Those skilled in the art will appreciate other ways of inserting the antibiotic into the balloon.

In general, in another aspect illustrated in Figure 38, the invention features a bone anchor implantation 210 device that has a spring element attached to the shaft within the balloon which retracts when the balloon contacts the bone anchor implantation site causing the bone anchor to perforate the balloon and implant in the bone. The spring element reduces the amount of force required to perforate the sheath and implant the bone anchor in the bone. The spring element 285 can be any type of spring which is elastically or plastically deformable. The spring element 285 has a first end 286 which is attached or grounded to the bone anchor mount 238 and a second floating end 287 which contacts the balloon. Preferably, the spring is a compression spring which is normally in an open position. A compression spring retracts when pressure is applied causing the point to puncture the balloon. As illustrated in Figure 38, the spring element 285 can be an open-coiled helical spring which surrounds the bone anchor 248. The spring mechanism 285 must have some minimum defection strength so as to prevent the bone anchor from puncturing the balloon prematurely during insertion but must have enough deflection strength to puncture the balloon when force is applied to the bone anchor implantation device at the desired location. The balloon 279 which covers the spring and the bone anchor has the same attributes as the balloon discussed above with respect to Figures 25-26a.

Another embodiment of the protective sheath is illustrated in Figure 26b. The bone anchor implantation device 210 as described above with respect to Figures 14-17 has a gelatin structure 290 which covers the bone anchor. The bone anchor implantation device 210 has a gelatin structure or caplet which is coupled to the bone anchor mount 238 and which encapsulates the bone anchor 248. The gelatin structure 290 protects the bone anchor 248 from contamination. As discussed above with reference to Figures 25-26a, the gelatin structure may be filled with an antibiotic. The device may also include a port for inserting antibiotic into the gelatin structure.

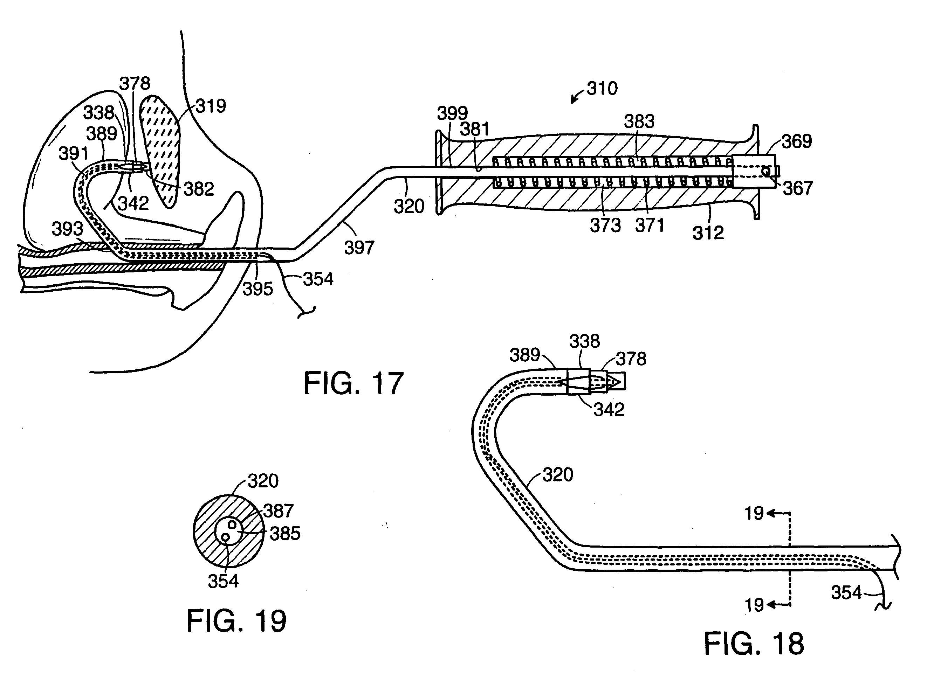

An alternative embodiment of the bone anchor implantation device 310 is shown in Figures 17-20 and Figure 27.

As illustrated in Figures 17 and 18, the shaft 320 has a generally straight proximal section 399, a first generally bent section 397, a generally straight median section 395, a second bent section 393, a generally curved section 391, and a distal generally straight section 389.

The straight proximal section 399 may be from about 7.62 cm (3.0 inches) to about 15.24 cm (6.0 inches) in length. Preferably, the straight proximal section 399 is from about 10.16 cm (4.0 inches) to about 12.7 cm (5.0 inches) in length. More preferably, the straight proximal section 399 is about 11.43 cm (4.5 inches) in length.

The first bent section 397 may be from about 2.54 cm (1.0 inches) to about 7.62 cm (3.0 inches) in length. Preferably, the first bent section 397 is from about 3.81 cm (1.5 inches) to about 6.35 cm (2.5 inches) in length. More preferably, the first bent section 397 is about 5.08 cm (2 inches) in length.

The first bent section 397 may bend at an angle of from about 35° to about 55° relative to the straight proximal section 399. Preferably, the first bent section 397 bends at an angle of from about 40° to about 50° relative to the straight proximal section 399. More preferably, the first bent section 397 bends at an angle of about 45° relative to the straight proximal section 399.

The straight median section 395 may be from about 5.08 cm (2 inches) to about 0.16 cm (4 inches) in length. Preferably, the straight median section 395 is from about 6.35 cm (2.5 inches) to about 8.89 cm (3.5 inches) in length. More preferably, the straight median section 395 is about 7.62 cm (3 inches) in length.

The second bent section 393 may be from about 1.27 cm (0.5 inches) to about 6.35 cm (2.5 inches) in length. Preferably, the second bent section 393 is from about 2.54 cm (1.0 inches) to about 5.08 cm (2.0 inches) in length. More preferably, the second bent section 393 is about 3.81 cm (1.5 inches) in length.

The second bent section 393 may bend at an angle of from about 125° to about 145° relative to the straight median section 395. Preferably, the second bent section 393 bends at an angle of from about 130° to about 140° relative to the straight median section 395. More preferably, the second bent section 393 bends at an angle of about 135° relative to the straight median section 395.

The curved section 391 may curve through an arc of from about 70° to about 110° with a radius from about 0.508 cm (0.2 inches) to about 1.524 cm (0.6 inches). Preferably, the curved section curves 391 through an arc of from about 80° to about 100° with a radius from about 0.762 cm (0.3 inches) to about 1.27 cm (0.5 inches). More preferably, the curved section 391 curves through an arc of about 90° with a radius of 0.16 cm (0.4 inches).

The distal straight section 389 may be from about 1.27 cm (0.5 inches) to about 2.286 cm (0.9 inches) in length. Preferably, the distal straight section 389 is from about 1.524 cm (0.6 inches) to about 2.032 cm (0.8 inches) in length. More preferably, the distal straight section 389 is about 1.778 cm (0.7 inches) in length.

The shaft 320 has a lumen extending therethrough. The lumen may have a diameter from about 0.076 cm (0.03 inches) to about 0.178 cm (0,07 inches) and the shaft 320 may have an outer diameter from about 0.508 cm (0.2 inches) to about 0.762 cm (0.3 inches). Preferably, the lumen has a diameter from about 0.102 cm (0.04 inches) to about 0.152 cm (0.06 inches) and the shaft 320 has an outer diameter from about 0.61 cm (0.24 inches) to about 0.66 cm (0.26 inches). More preferably, the lumen has a diameter of about 0.127 cm (0.05 inches) and the shaft 320 has an outer diameter of about 0.635 cm (0.250 inches).

Preferably, the shaft 320 has an insert 387 therein with a lumen 385 extending therethrough as best illustrated in the cross section of Figure 19. The insert 387 may be made of a variety of materials such as stainless steel or plastic.

The insert 3 87 has an outer diameter approximately that of the diameter of the lumen in the shaft such that the insert 387 fits snugly within the lumen of the shaft. The insert 387 may have an outer diameter from about 0.508 cm (0.2 inches) to about 0.762 cm (0.3 inches). Preferably, the insert 387 has an outer diameter from about 0.533 cm (0.21 inches) to about 0.686 (0.27 inches). More preferably, the insert 387 has an outer diameter of about 0.584 cm (0.23 inches).

The insert 387 has a lumen 385 extending therethrough having a diameter large enough to accommodate a suture 354. The diameter of the lumen 385 may be from about 0.051 cm (0.02 inches) to about 0.254 cm (0.100 inches). Preferably, the diameter of the lumen 385 is from about 0.102 cm (0.04 inches) to about 0.203 cm (0.08 inches). More preferably, the diameter of the lumen 385 is about 0.152 cm (0.06 inches).

As illustrated in Figures 17, 18 and 20, the shaft 3 20 has a bore therein which is large enough to permit the suture 354 to exit from the shaft 320. In the embodiment shown in Figures 17, 18 and 20, the bore is located in the straight median section 395 at a position in which it is located outside of the patient's body when the bone anchor 348 has been inserted into the patient's bone. However, those skilled in the art will appreciate that the bore may be located in other locations such as the first bent section 397.

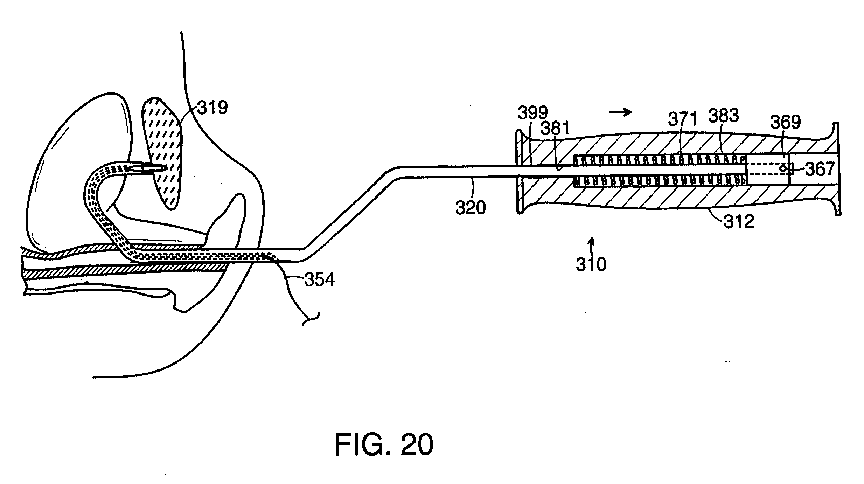

As illustrated in Figures 17 and 20, the shaft 320 extends through a lumen 3 83 in the handle 312. The lumen 383 has a narrow distal section 381 having a diameter slightly larger than the outer diameter of the shaft 320 and a wide proximal section 373 adapted to receive a spring 371.

The shaft 320 passes through the interior of the spring 371 as depicted in Figures 17 and 20. The distal end of the spring 371 contacts the distal end of the wider proximal section 373 of the lumen. The proximal end of the spring contacts a plug 369. The plug 369 has a lumen through which the shaft 320 passes and a bore adapted to receive a screw 367. The screw 367 passes through the bore in the plug 369 and a bore in the shaft 320 which is aligned with the bore in the plug, thereby securing the shaft 320 to the plug 369.

The resistance of the spring 371 is selected to be equal to the force with which the bone anchor 348 is to be driven into the bone. For example, where the bone anchor 348 is to be driven into the bone by applying 9.072 kg (20 pounds) of force, the spring 371 is a 9.072 kg (20 pound) spring. The spring indicates when the desired amount of force has been applied because the user can sense when the spring has been completely compressed.

The spring 371 may have a resistance of from about 2.27 (5) to about 15.88 kg (35 pounds). Preferably, the spring 371 has a resistance from about 6.8 to 11.34 kg (15 to 25 pounds). More preferably, the spring 371 has a resistance of 9.072 kg (20 pounds).

Those skilled in the art will appreciate that the anchor implantation device shown in Figures 13-16 may also be adapted to include a force indicating spring in the handle.

As illustrated in Figures 17, 18 and 20, a bone anchor mount 338 and a protective sheath 378 as described above with respect to the embodiment of Figures 13-16 are attached to the end of the distal straight section 389.

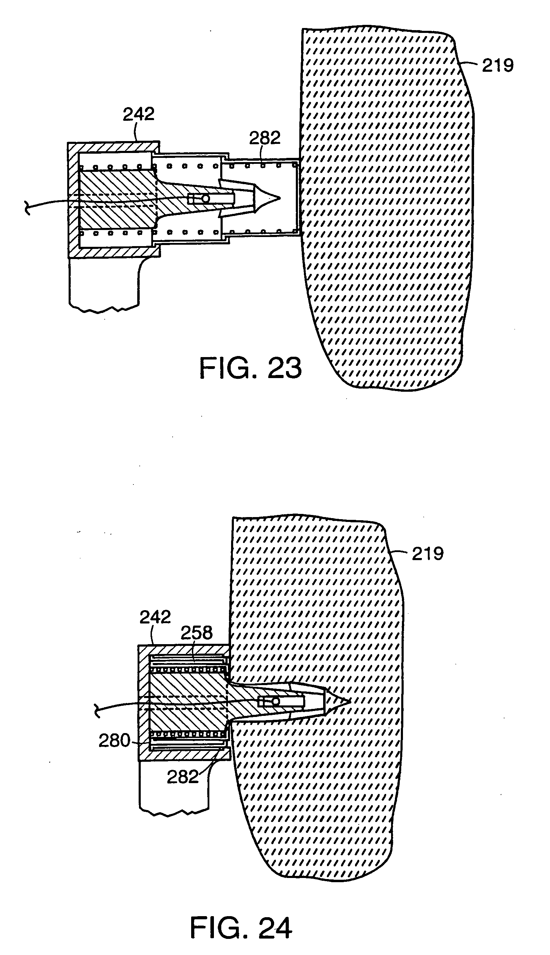

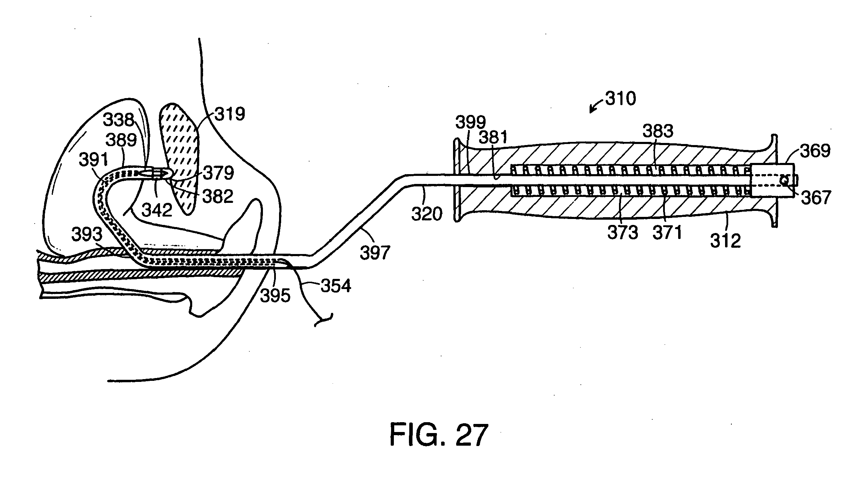

In an alternate embodiment, illustrated in Figure 27, the protective sheath on the bone anchor implantation device 310 is a balloon 279 as described above with respect to the embodiments of Figures 25 and 26a. The balloon is attached to the distal straight section 389 of the bone anchor implantation device 310 as described above with respect to the embodiment of Figures 24 and 25. The device may further comprise a spring element as discussed above with respect to Figure 38.

The hooked bone anchor implantation devices 210, 310 are used as follows. An incision in the anterior vaginal wall is made as described above. The site for bone anchor implantation is located by palpation as described above.

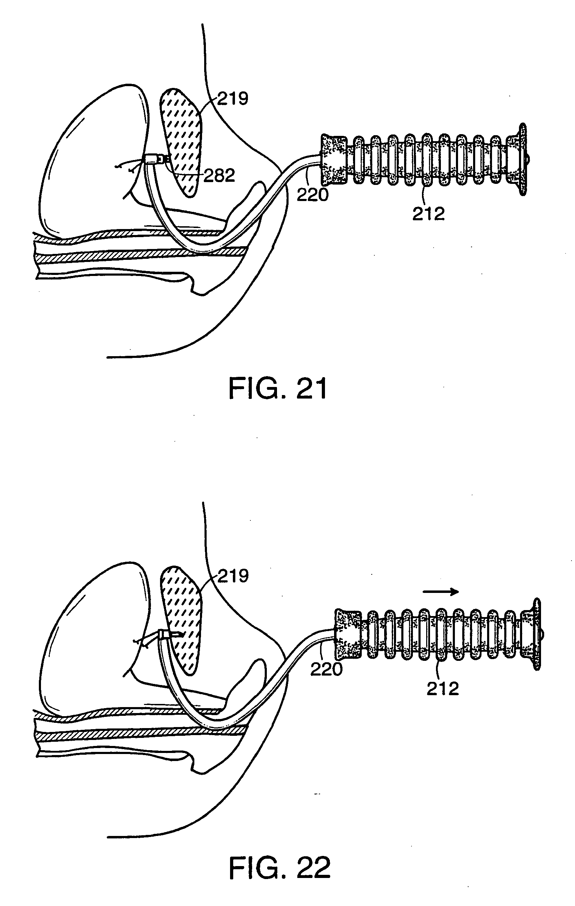

The hooked bone anchor implantation device 210, 310 is inserted into the vagina as shown in Figures 17 and 21 with the patient in the lithotomy position and the surgeon located between the patient's legs. The shaft 220, 320 is inserted through the incision and the protective sheath 278, 378 is positioned such that the proximal end of the second telescoping cylinder 282, 382 contacts the pubic bone 219, 319 as shown in Figures 17, 21 and 23. At this time, the first and second telescoping cylinders 280, 380, 282, 382 are biased to a position in which they extend from the outer cylinder 242, 342 to cover the bone anchor. The bone anchor is inserted into the bone by applying a retrograde force to the bone anchor. The retrograde force can be applied in a number of ways as will be apparent to one of skill in the art. Preferably, the bone anchor is implanted by pulling the handle. For example, the handle may be pulled in a retrograde direction (toward the user) to implant the anchor as shown in Figures 20 and 22. As the device is pulled in a retrograde motion, the first and second telescoping cylinders 280, 282, 380, 382 retract inside the cavity 258, 358 of the outer cylinder as shown in Figures 20, 22 and 24 and the bone anchor 248, 348 is driven into the pubic bone 219, 319. Because the patient's body weight provides an opposing force, the user need only apply a small amount of force, such as 4.54-9.07 kg (10-20 pounds), in order to drive the bone anchor 248, 348 into the bone 219, 319. The device 210, 310 is then pushed away from the implanted anchor to disengage the device from the anchor. The device is then removed from the vagina, leaving the bone anchor 248, 319 in the bone 219, 319 with the suture extending therefrom. The bladder neck is then compressed, suspended or stabilized using the suture(s) extending from the bone anchor(s) as described above.

As shown in Figure 20, in the device 310 having a spring 371 inside the handle 312, the spring 371 is compressed when the handle is pulled in a retrograde direction to drive the bone anchor into the bone. In this embodiment, the user can detect when the spring 371 has been completely compressed, or compressed by a predetermined amount, indicating that the desired amount of force for driving the bone anchor into the bone has been applied.

The hooked bone anchor implantation device with balloon illustrated in Figures 25-27 is used as follows. An incision in the anterior vaginal wall is made as described above. The site for bone anchor implantation is located by palpation of the urethra, pubic symphsis or other anatomical landmark or other techniques known by those skilled in the art.

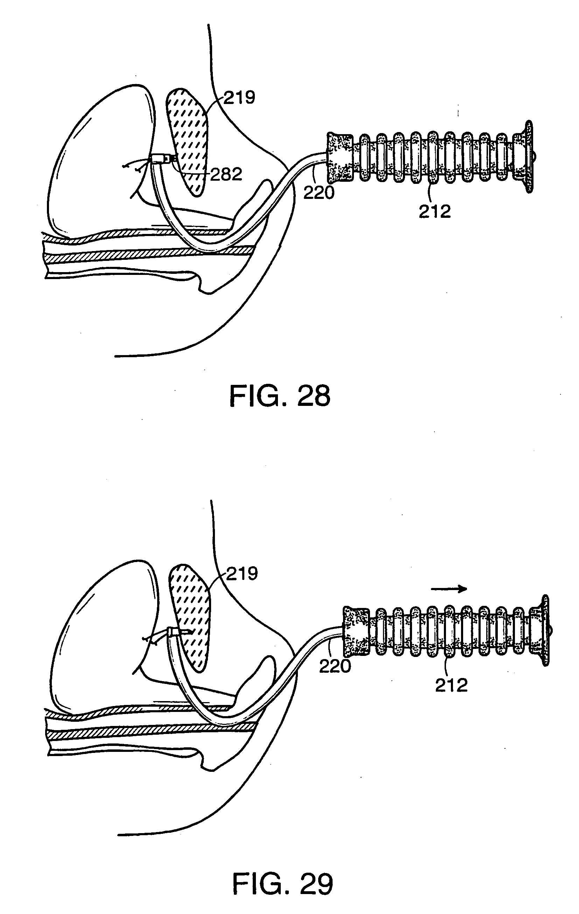

The hooked bone anchor implantation device with a balloon or gelatin structure 210, 310 is inserted into the vagina as shown in Figures 27 and 28 with the patient in the lithotomy position and the surgeon located between the patient's legs. The shaft 220, 320 is inserted through the incision and the balloon or gelatin structure 279, 379 is positioned such that the balloon or gelatin structure 279 contacts the pubic bone 219, 319 as shown in Figures 27 and 28. The bone anchor is inserted into the bone by applying a retrograde force to the bone anchor 238,348. The force is transmitted through the bone anchor implantation device 210, 310 to the bone anchor 248, 348 and causes the balloon or gelatin structure 279, 379 to press against the pubic bone 219, 319. The application of additional force causes the bone anchor point to puncture the balloon or gelatin structure 279, 379 and drives the bone anchor 248, 348 into the pubic bone 219, 319. Because the patient's body weight provides an opposing force, the user need only apply a small amount of force, such as 4.54 to 9.07 kg (10-20 pounds), in order to drive the bone anchor 248, 348 into the bone 219,319 In one embodiment (not shown), the retrograde force retracts a spring element attached to the shaft within the balloon which causes the bone anchor to perforate the sheath and implant into the bone. In a preferred embodiment, an antibiotic is inserted into the balloon or gelatin structure and is released when the sheath is punctured. The antibiotic may be inserted into the balloon or gelatin structure via a port which extends from one end of the shaft to the other end of the shaft into the balloon. The device 210, 310 is then pushed away from the implanted anchor to disengage the device from the anchor. The device is then removed from the vagina, leaving the bone anchor 248, 319 in the bone 219, 319 with the suture extending therefrom. The bladder neck is then compressed, suspended or stabilized using the suture(s) extending from the bone anchor(s) as described above.

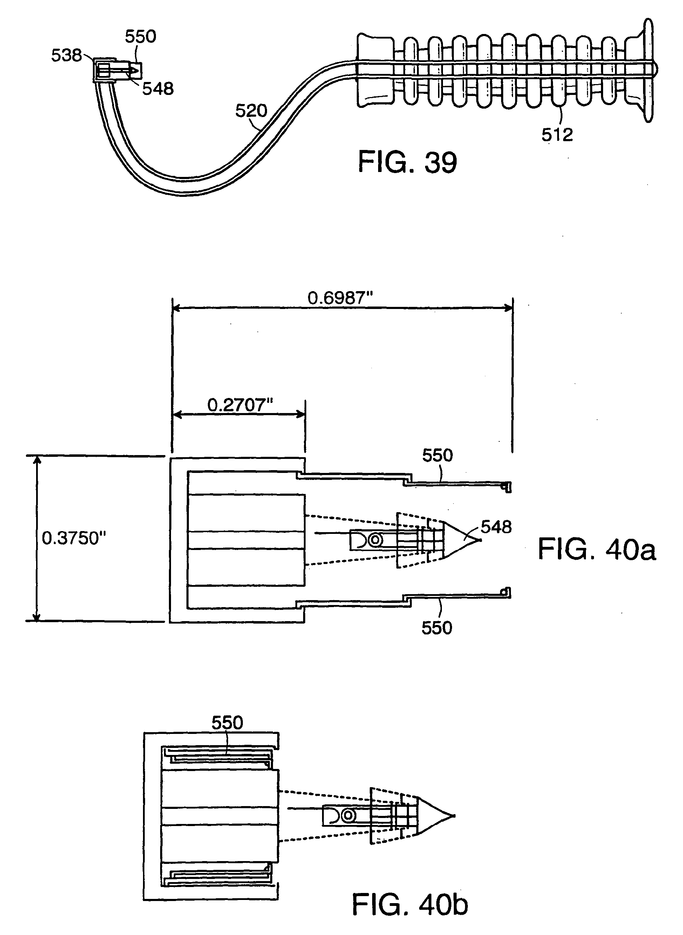

Other embodiments of the invention, particularly various types of protective sheaths which prevent premature insertion, are illustrated in Figures 39-45.

Figures 39-40b illustrate a bone anchor implantation device with a telescoping sheath which retracts upon insertion. As discussed above with reference to Figures 13-16, the bone anchor implantation device with telescoping sheath comprises a handle 512, a hooked shaped shaft 520 secured to the handle 212, a bone anchor mount 538 adapted to releasably engage a bone anchor 548 and attached at the distal end of the shaft 520, and a telescoping sheath S50 which attaches to the bone anchor mount 538 and covers the bone anchor 548. Figure 40a depicts the telescoping sheath 550 in an extended or open position covering the bone anchor 548. Figure 40B illustrates the telescoping sheath 550 in a retracted position. Details regarding the telescoping sheath are discussed above with reference to Figures 13-16. A spring biases the telescoping sheath 550 between extended and retracted positions.

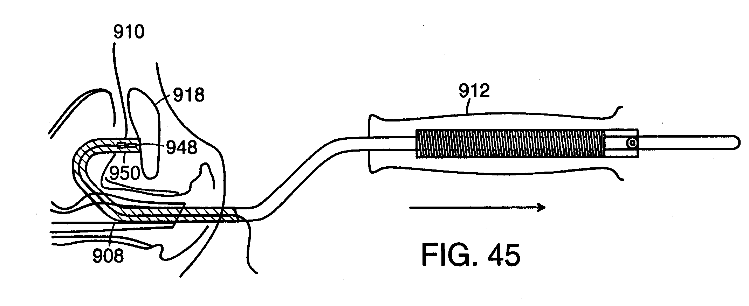

As illustrated in Figure 45 the bone anchor implantation device with telescoping sheath shown in Figure 39 is inserted into the vagina 908 and positioned so that the telescoping sheath 950 contacts the pubic bone 918. The bone anchor 948 is then inserted by applying a retrograde force to the bone anchor 948 by pulling the handle 912 of the device in a retrograde direction. There is an annular shoulder 910 on the bone anchor 948 which acts as a depth stop to ensure adequate penetration. Further details regarding the method of inserting the bone anchor are described above with reference to Figures 17 and 21.

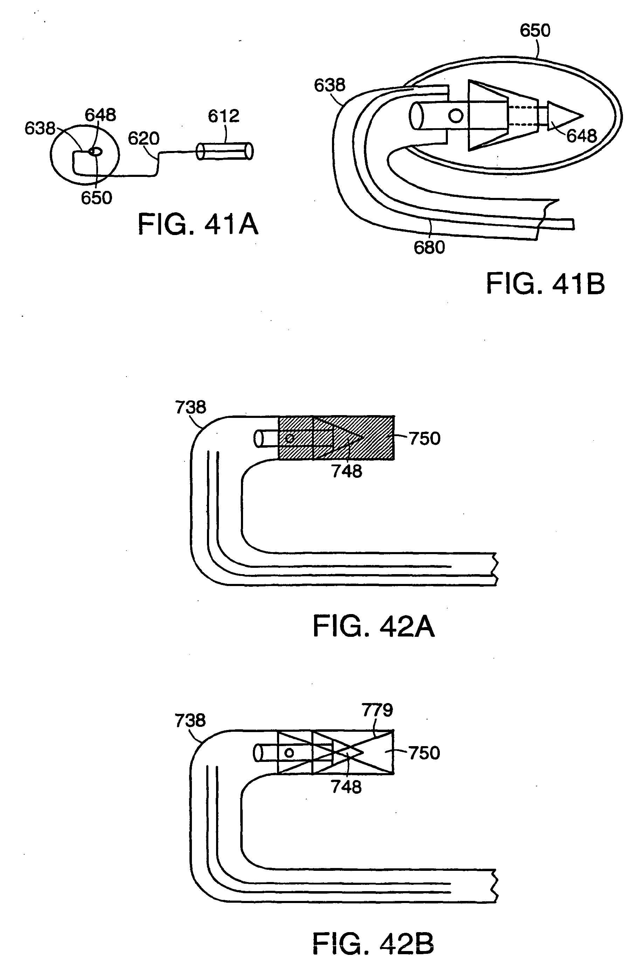

Figures 41a and 41b illustrate a hooked-shaped bone anchor implantation device which a balloon 650 covering the bone anchor 648. The device comprises a handle 612, a hooked-shaped shaft secured to the handle 612, a bone anchor mount 638 attached to the distal end of the shaft and adapted to releasably engage a bone anchor 648, and a balloon 650 which covers the bone anchor 648. Features of the balloon sheath are discussed above with reference to Figures 25 and 26a. The balloon 650 may be filled with one or more antibiotics to combat infection at he implantation site. The device may also comprise a port 680 for inserting antibiotics into the balloon. The balloon is perfed away upon implantation.

Figures 42a and 42b illustrate a bone anchor implantation device having a latex sheath 750 with a spring element 779 which covers the bone anchor 748. As described above with reference to Figure 38, the spring element can be any type of spring which is elastically or plastically deformable. Preferably the spring is a compression spring such as a coiled helical spring which surrounds the bone anchor and which retracts when pressure is applied. The latex sheath can be hermetically sealed to the bone anchor implantation device. The sheath may also be filled with one or more antibiotics to prevent infection at the implantation site. The latex sheath perfs away upon implantation.

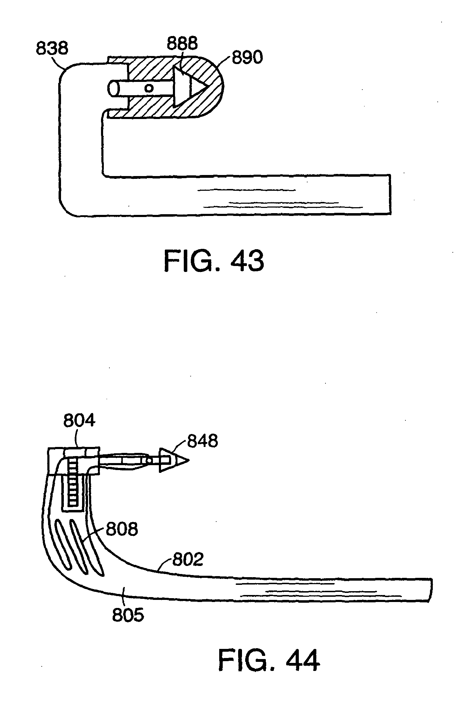

Figure 43 illustrates a bone anchor implantation device with gelatin structure or a capsule 890 that covers the bone anchor 888. The capsule 890 can be coupled to the bone anchor mount 838. The capsule 890 can be made of biodegradable materials such as those described above with reference to the balloon sheath illustrated in Figures 25 and 26a. Preferably the caplet is made of gelatin. The bone anchor 888 perfs the capsule 890 and any remaining capsule particles are absorbed or metabolized.

Figure 44 illustrates a bone anchor implantation device that has a sheath 802 with a lumen 805 that a diameter large enough to accommodate one or more sutures 808 which are preattached to the bone anchor 848. The sheath 802 protect the sutures 808 from contamination. Further details regarding a shaft with a lumen that accommodates one or more sutures are discussed above with respect to Figures 17-20. The bone anchor 848 with attached sutures 808 has a screw or locking device 804 which secures the bone anchor 848 to the bone anchor implantation device. The locking mechanism 804 prevents accidental insertion of the bone anchor 848 into tissue.

Although this invention has been described in terms of certain preferred embodiments, other embodiments which will be apparent to those of ordinary skills in the art in view of the disclosure herein are also within the scope of this invention. Accordingly, the scope of the invention is intended to be defined only by reference to the appended claims.

## Claims

1. A bone anchor implantation device (10, 210, 310), comprising:
a hook-shaped shaft (20, 220, 320, 520) having a first end and a second end;
a bone anchor (22, 122, 248, 348, 548, 648, 748) releasably engaged to one end of the shaft;
a protective sheath (58, 278, 378) for encapsulating the bone anchor prior to implantation, and
wherein the protective sheath comprises a balloon (279, 379, 650).

2. The device of Claim 1, wherein the protective sheath comprises:
at least one telescoping cylinder;
a first telescoping cylinder (280, 380) having a first proximal end, a distal end and a lumen (286) extending therethrough, a first shoulder (288) at said distal end of said first telescoping cylinder and a second shoulder at said proximal end of said first telescopic cylinder;
a second telescoping cylinder (282, 382) having a proximal end, a distal end and a lumen (296) extending therethrough, a first shoulder at said distal end of said second telescoping cylinder and a second shoulder at said proximal end of said second telescoping cylinder; and
a spring (284), wherein said first shoulder of said first telescoping cylinder (280, 380) engages said shoulder of said outer cylinder (242, 342), said second shoulder of said first telescoping cylinder (280, 380) engages said first shoulder of said second telescoping cylinder (282, 382), and said spring (284) is disposed between said second shoulder of said second telescoping cylinder and said outer cylinder, whereby said first and second telescoping cylinders are moveable between a first position in which they are extended from said outer cylinder to cover said bone anchor and a second position in which they are retracted in said cavity (258, 358) of said outer cylinder to expose said bone anchor.

3. The device of Claim 1 wherein the protective sheath comprises a gelatin structure (290).

4. The device of Claims 1 to 3 wherein said balloon (279, 379, 650) is perforatable by the bone anchor as the bone anchor is pressed into a bone.

5. The device of any foregoing claim wherein said balloon (279, 379, 650) is hermetically sealed around the bone anchor.

6. The device of any foregoing claim wherein said balloon (279, 379, 650) comprises a material selected from the group consisting of plastic, thermoplastic, elastromers, PET, PETG, rubber, vinyl, latex and silicone, the material preferably being latex.

7. The device of Claims 1 to 5 wherein said balloon (279, 379, 650) comprises a biodegradable material.

8. The device of Claim 7 wherein said biodegradable material comprises a polymer, preferably a synthetic polymer.

9. The device of Claim 8 wherein said polymer is selected from the group consisting of polyglycolic acid (PGA), polylactic acid (PLA), poly (dioxanone) (PDO), poly (1-lactide) (LPLA), poly (dl-lactide) (DLPLA), poly (glycolide-co-trimethylene carbonate) (PGA-TMC), poly (1-lactide-co-glycolide) (PGA-LPLA), poly (dl-lactide-co-glycolide) (PGA-DLPLA), poly (1-lactide-co-dl-lactide) (LPLA-DLPLA), poly (glycolide-co-trimethylene carbonate-co-dioxanone) (PDO-PGA-TMC), poly (ε-caprolactone), poly (dioxanone) (a polyether-ester), poly (lactide-co-glycotide), poly (SA-HDA anhydride), poly (orthoester), polyglyconate.

10. The device of any foregoing claim wherein said protective sheath contains an antibiotic.

11. The device of any foregoing claim further comprising a port (280, 680) which extends from the first end to the second end of said shaft into the protective sheath.

12. The device of Claim 10 wherein said antibiotic is released when the protective sheath is perforated.

13. The device of Claim 10 wherein said antibiotic is selected from the group consisting of nafcillin, aminogylcoside, ciprofloxin, clindamcin, piperacillin/tazobactum, ampicillin/sulbactum, aminoglcoside, vancomycin, cephalosporin, TMP/SMX, ampicillin, gentaminicin, tobramcycin and ciprofloxacin.

14. The device of any foregoing claim further comprising a spring element (285) attached to the shaft within to the balloon which retracts when the balloon is pressed against the bone by the shaft causing the bone anchor to perforate the sheath.

15. The device of Claim 14 wherein the spring element (285) comprises an open-coiled helical spring which surrounds the bone anchor.

16. The device of Claim 1 wherein one or more sutures (808) are coupled to the bone anchor and wherein the shaft includes a hollow section in which the sutures are disposed.

## Patentansprüche

1. KnochenankerImplantierungsvorrichtung (10, 210, 310), umfassend:
einen hakenförmigen Schaft (20, 220, 320, 520) mit einem ersten Ende und einem zweiten Ende;
einen Knochenanker (22, 122, 248, 348, 548, 648, 748), welcher lösbar mit einem Ende des Schafts in Eingriff gelangt;
eine Schutzhülle (58, 278, 378) für das Einkapseln des Knochenankers vor der Implantierung, und
wobei die Schutzhülle einen Ballon (279, 379, 650) umfasst.

2. Vorrichtung nach Anspruch 1, wobei die Schutzhülle umfasst:
mindestens einen teleskopartigen Zylinder;
einen ersten teleskopartigen Zylinder (280, 380) mit einem ersten proximalen Ende, einem distalen Ende und einem Hohlraum (296), welcher sich durch diesen hindurch erstreckt, einer ersten Schulter (288) an dem distalen Ende des ersten teleskopartigen Zylinders und einer zweiten Schulter an dem proximalen Ende des ersten teleskopartigen Zylinders;
einen zweiten teleskopartigen Zylinder (282, 382) mit einem proximalen Ende, einem distalen Ende und einem Hohlraum (296), welcher sich durch diesen hindurch erstreckt, einer ersten Schulter an dem distalen Ende des zweiten teleskopartigen Zylinders und einer zweiten Schulter an dem proximalen Ende des zweiten teleskopartigen Zylinders; und
eine Feder (284), wobei die erste Schulter des ersten teleskopartigen Zylinders (280, 380) mit der Schulter des äußeren Zylinders (242, 342) in Eingriff gelangt, die zweite Schulter des ersten teleskopartigen Zylinders (280, 380) mit der ersten Schulter des zweiten teleskopartigen Zylinders (282, 382) in Eingriff gelangt und die Feder (284) zwischen der zweiten Schulter des zweiten teleskopartigen Zylinders und dem äußeren Zylinder angeordnet ist, wodurch der erste und der zweite teleskopartige Zylinder zwischen einer ersten Position, in welcher sie ausgehend von dem äußeren Zylinder ausgezogen sind, um den Knochenanker zu bedecken, und einer zweiten Position, in welcher sie in den Hohlraum (258, 358) des äußeren Zylinders zurückgeschoben sind, um den Knochenanker freizulegen, beweglich sind.

3. Vorrichtung nach Anspruch 1, wobei die Schutzhülle eine Gelatinestruktur (290) umfasst.

4. Vorrichtung nach den Ansprüchen 1 bis 3, wobei der Ballon (279, 379, 650) durch den Knochenanker durchbohrbar ist, wenn der Knochenanker in einen Knochen gedrückt wird.

5. Vorrichtung nach einem der vorangegangenen Ansprüche, wobei der Ballon (279, 379, 650) um den Knochenanker herum hermetisch abgedichtet ist.

6. Vorrichtung nach einem der vorangegangenen Ansprüche, wobei der Ballon (279, 379, 650) ein Material umfasst, welches aus der Gruppe bestehend aus Kunststoff, thermoplastischen Polymeren, Elastomeren, PET, PETG, Gummi, Vinyl, Latex und Silicon ausgewählt wird, umfasst, wobei das Material vorzugsweise Latex ist.

7. Vorrichtung nach den Ansprüchen 1 bis 5, wobei der Ballon (279, 379, 650) ein biologisch abbaubares Material umfasst.

8. Vorrichtung nach Anspruch 7, wobei das biologisch abbaubare Material ein Polymer, vorzugsweise ein synthetisches Polymer umfasst.

9. Vorrichtung nach Anspruch 8, wobei das Polymer aus der Gruppe bestehend aus Polyglycolsäure (PGA), Polymilchsäure (PLA), Poly(dioxanon) (PDO), Poly(1-lactid) (LPLA), Poly(dl-lactid) (DLPLA), Poly(glycolid-co-trimethylencarbonat) (PGA-TMC), Poly(1-lactid-co-glycolid) (PGA-LPLA), Poly(dl-lactid-co-glycolid) (PGA-DLPLA), Poly(l-lactid-co-dl-lactid) (LPLA-DLPLA), Poty(giycolid-co-trimethylencarbonat-co-dioxanon) (PDO-PGA-TMC), Poly(s-caprolacton), Poly(dioxanon) (ein Polyether-Ester), Poly(lactid-co-glycotid). Poly(SA-HDA-Anhydrid), Poly(orthoester), Polyglyconat ausgewählt wird.

10. Vorrichtung nach einem der vorangegangenen Ansprüche, wobei die Schutzhülle ein Antibiotikum umfasst.

11. Vorrichtung nach einem der vorangegangenen Ansprüche, die des Weiteren eine Durchgangsöffnung (280, 680) umfasst, die sich von dem ersten Ende bis zu dem zweiten Ende des Schafts in die Schutzhülle erstreckt.

12. Vorrichtung nach Anspruch 10, wobei das Antibiotikum freigesetzt wird, wenn die Schutzhülle durchbohrt wird.

13. Vorrichtung nach Anspruch 10, wobei das Antibiotikum aus der Gruppe bestehend aus Nafcillin, Aminoglycosid, Ciprofloxin, Clindamycin, Piperacillin/Tazobactum, Ampicillin/Sulbactum, Aminoglcosid, Vancomycin, Cephalosporin, TMP/SMX, Ampicillin, Gentamycin, Tobramycin und Ciprofloxacin ausgewählt wird.

14. Vorrichtung nach einem der vorangegangenen Ansprüche, welche des Weiteren ein Federelement (285) umfasst, welches an dem Schaft innerhalb des Ballons befestigt ist, und sich zurückzieht, wenn der Ballon durch den Schaft gegen den Knochen gedrückt wird, wodurch bewirkt wird, dass der Knochenanker die Hülle durchbohrt.

15. Vorrichtung nach Anspruch 14, wobei das Federelement (285) eine Schraubenfeder mit offener Windung ist, die den Knochenanker umgibt.

16. Vorrichtung nach Anspruch 1, wobei ein oder mehrere Nahtmaterialien (808) mit dem Knochenanker verbunden sind und wobei der Schaft einen hohlen Abschnitt umfasst, in welchem die Nahtmaterialien angeordnet sind.

## Revendications

1. Dispositif d'implantation d'ancrage osseux (10, 210, 310), comprenant :
une tige recourbée (20, 220, 320, 520) ayant une première extrémité et une seconde extrémité ;
un ancrage osseux (22, 122, 248, 348, 548, 648, 748) s'enclenchant de manière amovible à une extrémité de la tige ;
une gaine de protection (58, 278, 378) pour encapsuler l'ancrage osseux avant l'implantation, et
dans lequel la gaine de protection comprend un ballonnet (279, 379, 650).

2. Dispositif selon la revendication 1, dans lequel la gaine de protection comprend :
au moins un cylindre télescopique ;
un premier cylindre télescopique (280, 380) ayant une première extrémité proximale, une extrémité distale et une lumière (286) traversant celui-ci, un premier épaulement (288) au niveau de ladite extrémité distale dudit premier cylindre télescopique et un second épaulement au niveau de ladite extrémité proximale dudit premier cylindre télescopique ;
un second cylindre télescopique (282, 382) ayant une extrémité proximale, une extrémité distale et une lumière (296) traversant celui-ci, un premier épaulement au niveau de ladite extrémité distale dudit second cylindre télescopique et un second épaulement au niveau de ladite extrémité proximale dudit second cylindre télescopique ; et
un ressort (284), ou ledit premier épaulement dudit premier cylindre télescopique (280, 380) s'enclenche audit épaulement dudit cylindre externe (242, 342), ledit second épaulement dudit premier cylindre télescopique (280, 380) s'enclenche audit premier épaulement dudit second cylindre télescopique (282, 382) et ledit ressort (284) est disposé entre ledit second épaulement dudit second cylindre télescopique et ledit cylindre externe, ce par quoi lesdits premier et second cylindres télescopiques peuvent se déplacer entre une première position dans laquelle ils s'étendent depuis ledit cylindre externe pour recouvrir ledit ancrage osseux et une seconde position dans laquelle ils sont rétractés dans ladite cavité (258, 358) dudit cylindre externe pour découvrir ledit ancrage osseux.

3. Dispositif selon la revendication 1, dans lequel la gaine de protection comprend une structure gélatineuse (290).

4. Dispositif selon les revendications 1 à 3, dans lequel ledit ballonnet (279, 379, 650) peut être perforé par l'ancrage osseux lorsque l'ancrage osseux est enfoncé dans un os.

5. Dispositif selon l'une quelconque des revendications précédentes, dans lequel ledit ballonnet (279, 379, 650) est hermétiquement fermé autour de l'ancrage osseux.

6. Dispositif selon l'une quelconque des revendications précédentes, dans lequel ledit ballonnet (279, 379, 650) comprend un matériau choisi dans le groupe constitué par des matières plastiques, des matières thermoplastiques, des élastomères, du PET, du PETG, du caoutchouc, du vinyle, du latex et de la silicone, le matériau étant de préférence du latex.

7. Dispositif selon les revendications 1 à 5, dans lequel ledit ballonnet (279, 379, 650) comprend un matériau biodégradable.

8. Dispositif selon la revendication 7, dans lequel ledit matériau biodégradable comprend un polymère, de préférence un polymère synthétique.

9. Dispositif selon la revendication 8, dans lequel ledit polymère est choisi dans le groupe constitué par l'acide polyglycolique (PGA), l'acide polylactique (PLA), la poly(dioxanone) (PDO), le poly(1-lactide) (LPLA), le poly(dl-lactide) (DLPLA), le poly(glycolide/co-carbonate de triméthylène) (PGA-TMC), le poly(1-lactide/co-glycolide) (PGA-LPLA), le poly(dl-lactide/co-glycolide) (PGA-DLPLA), le poly(1-lactide/co-dl-lactide) (LPLA-DLPLA), le poly(glycolide/co-carbonate de triméthylène/dioxanone) (PDO-PGA-TMC), la poly(ε-caprolactone), la poly(dioxanone) (a-polyéther-ester), le poly(lactide/co-glycotide), le poly(SA-HDA anhydride), le poly(orthoester), le polyglyconate.

10. Dispositif selon l'une quelconque des revendications précédentes, dans lequel ladite gaine de protection contient un antibiotique.

11. Dispositif selon l'une quelconque des revendications précédentes, comprenant en outre un orifice (280, 680) qui s'étend de la première extrémité à la seconde extrémité de ladite tige dans la gaine de protection.

12. Dispositif selon la revendication 10, dans lequel ledit antibiotique est libéré lorsque la gaine de protection est perforée.

13. Dispositif selon la revendication 10, dans lequel ledit antibiotique est choisi dans le groupe constitué par la nafcilline, l'aminoglycoside, la ciprofloxine, la clindamicine, le pipéracilline/tazobactam, l'ampicilline/sulbactam, l'aminoglycoside, la vancomycine, la céphalosporine, le TMP/SMX, l'ampicilline, la gentamicine, la tobramycine et la ciprofloxacine.

14. Dispositif selon l'une quelconque des revendications précédentes, comprenant en outre un élément élastique (285) fixé à la tige à l'intérieur jusqu'au ballonnet qui se rétracte lorsque le ballonnet est appuyé contre l'os par la tige, ce qui entraîne la perforation de la gaine par l'ancrage osseux.

15. Dispositif selon la revendication 14, dans lequel l'élément élastique (285) comprend un ressort hélicoïdal à spires non jointives qui entoure l'ancrage osseux.

16. Dispositif selon la revendication 1, dans lequel une ou plusieurs suture(s) (808) est/sont couplée(s) à l'ancrage osseux et dans lequel la tige comprend une section creuse dans lequel sont disposées les sutures.
